(19) Europäisches Patentamt
European Patent Office
Office européen des brevets

(11) EP 4 732 906 A2

(12) EUROPEAN PATENT APPLICATION

(43) Date of publication:
29.04.2026 Bulletin 2026/18

(21) Application number: 26155310.1

(22) Date of filing: 14.02.2023

(51) International Patent Classification (IPC):
*A61P 31/14* (2006.01)

(52) Cooperative Patent Classification (CPC):
A61K 9/0019; A61K 9/08; A61K 39/12;
A61P 31/14; A61K 2039/5254; C12N 2770/24134

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR

(30) Priority: 15.02.2022 US 202263310437 P
22.03.2022 EP 22163647

(62) Document number(s) of the earlier application(s) in
accordance with Art. 76 EPC:
23717773.8 / 4 479 025

(71) Applicant: Takeda Vaccines, Inc.
Cambridge, MA 02139 (US)

(72) Inventors:
• BRONSON, Sean
Cambridge, 02139 (US)

• SHOEMAKER, Scott
Cambridge, 02139 (US)

(74) Representative: Maiwald GmbH
Elisenhof
Elisenstraße 3
80335 München (DE)

Remarks:
•The complete document including Reference
Table(s) and the Sequence Listing(s) can be
downloaded from the EPO website
•This application was filed on 30-01-2026 as a
divisional application to the application mentioned
under INID code 62.

(54) **DENGUE VACCINE BATCH MIXING PROCESS**

(57) The present invention relates to a batch mixing process for preparing a liquid pharmaceutical composition (LPC) comprising at least one biological active agent and at least one adjustable excipient, wherein the at least one biological active agent has a target concentration in the LPC ($^T[A_i]_{LPC}$) and the at least one adjustable excipient has a target concentration in the LPC ($^T[E_x]_{LPC}$)

Figure 1:

EP 4 732 906 A2

## Description

### Cross Reference to Related Applications

The present application claims the benefit of, and priority to U.S. provisional application Number 63/310,437 filed on 15 February 2022, the entire contents of which is incorporated herein by reference in its entirety.

### SEQUENCE LISTING

This application contains a Sequence Listing which is submitted herewith in electronically readable XML ST.26 format.

### FIELD OF THE INVENTION

The present invention relates to a process for accurately controlling the concentration of at least one excipient and the concentration of at least one biological active agent in a liquid pharmaceutical composition. In particular, the present invention relates to a process for accurately controlling the concentration of at least one excipient and of at least one biological active agent in a liquid vaccine composition, such as e.g. a liquid dengue vaccine composition.

### BACKGROUND OF THE INVENTION

Certain pharmaceutical compositions, such as e.g. vaccines, comprise biological active agents, which are synthesized from biological/living sources (e.g. human, animal, plant, fungal, or microbial sources). In particular, these biological active agents may be derived from tissue cultures of human or animal cell lines (e.g. Vero cell lines). The concentration of biological active agent yielded by different batches/growth cycles of the living sources (in particular cell lines) can vary significantly, thus providing initial liquid drug substances (LDS) from the living sources with significantly varying concentrations of active agent. It is however necessary to ensure that the final pharmaceutical compositions, such as e.g. the vaccine, comprise an appropriate predefined dose of the biological active agent which does not vary significantly from batch to batch. At the same time one or more excipients need to be present in certain concentrations in the final composition to form a stable formulation. Said excipients may or may not be present at all in the initial liquid drug substance (LDS) or may or may not be present in the final concentration in the initial liquid drug substance (LDS). Such excipients are considered as adjustable excipients. Final pharmaceutical compositions according to this disclosure are initially liquid but may subsequently be dried, e.g. by lyophilization.

Thus manufacturing the final liquid pharmaceutical composition (LPC) from the initial liquid drug substance (LDS) requires adjusting several concentrations and in particular requires adjusting the concentration of the active agent based on significantly varying source concentrations. This leads to a highly variable batch mixing process. There is thus a need for an effective batch mixing process that minimizes process variables (batch to batch variables) despite batch to batch variable active agent starting materials.

### OBJECTS AND SUMMARY

It is an object of the present invention to provide a batch mixing process for preparing a liquid pharmaceutical composition (LPC) with a target concentration of at least one biological active agent and a target concentration of at least one adjustable excipient, wherein the LPC is prepared from one or more liquid drug substances (LDS) with a concentration of one biological active agent that may vary from batch to batch.

The present invention also relates to pharmaceutical compositions obtained or obtainable by said process and to the use of such pharmaceutical compositions, in a method of treatment or prevention.

The above objects are achieved by embodiments of the present invention as described and claimed herein.

The present invention is, therefore, directed to a batch mixing process for preparing a liquid pharmaceutical composition (LPC) comprising at least one biological active agent and at least one adjustable excipient, wherein the at least one biological active agent has a target concentration in the LPC ($^{T}[A_i]_{LPC}$) and the at least one adjustable excipient has a target concentration in the LPC ($^{T}[E_x]_{LPC}$), the process comprising at least two steps:

step 1: providing

a variable excipient liquid (VEL) comprising the at least one adjustable excipient at a predefined concentration ($^{P}[E_x]_{VEL}$),

one or more liquid drug substances (LDS) comprising one biological active agent at a variable concentration ($^V[A_i]_{LDS}$) and the at least one adjustable excipient at a predefined concentration ($^P[E_x]_{LDS}$), and

a fixed excipient liquid (FEL) comprising the at least one adjustable excipient at a predefined concentration ($^P[E_x]_{FEL}$), wherein the predefined concentration of each of the adjustable excipients is defined in accordance with equation 1:

$$^P[E_x]_{VEL} = {}^P[E_x]_{LDS} \neq {}^P[E_x]_{FEL,} \qquad (1)$$

wherein $^P[E_x]_{LDS}$ is predefined by the process of manufacture of LDS and $^P[E_x]_{FEL}$ is further defined by the conditions of step 2

step 2: combining

all of the LDSs each at a variable volume ($^VV_{LDS(i)}$),

the VEL at a variable volume ($^VV_{VEL}$), and

the FEL at a predefined volume ($^PV_{FEL}$),

to form the LPC with a target volume ($^TV_{LPC}$) and with a target concentration of all of the biological active agents and all of the adjustable excipients, wherein

the variable volume of each LDS is defined according to equation 2:

$$^vV_{LDS(i)} = \frac{^T[A_i]_{LPC}}{^V[A_i]_{LDS}} \times {}^TV_{LPC}, \qquad (2)$$

the combined total volume of all LDSs ($^VV_{LDS(total)}=\sum_{i=1}^{\infty} V_{LDS(i)}$) and the VEL is predefined ($^PV_{(VEL+LDS)}$), and the variable volume of the VEL is defined according to equation 3:

$$^vV_{VEL} = {}^pV_{(VEL+LDS)} - {}^vV_{LDS(total)}, \qquad (3)$$

and the predefined volume of the FEL ($^PV_{FEL}$) is defined according to equation 4:

$$^pV_{FEL} = {}^TV_{LPC} - {}^pV_{(VEL+LDS)}, \qquad (4)$$

and the pre-defined concentration of each of the adjustable excipients in the FEL is defined according to equation 5:

$$^p[E_x]_{FEL} = \frac{^T[E_x]_{LPC} \times {}^TV_{LPC} - {}^pV_{(VEL+LDS)} \times {}^p[E_x]_{VEL}}{^pV_{FEL}} \qquad (5)$$

In one embodiment, the target concentration of the at least one biological active agent in the LPC ($^T[A_i]_{LPC}$) and the target concentration of the at least one adjustable excipient in the LPC ($^T[E_x]_{LPC}$) are predefined. The ($^T[A_i]_{LPC}$) and ($^T[E_x]_{LPC}$) of the LPC do not change from batch to batch, i.e. they are constant. They are chosen at appropriate target values for the desired LPC.

It is in the nature of the invention that the concentration of the at least one adjustable excipient in the VEL ($^P[E_x]_{VEL}$), LDS ($^P[E_x]_{VEL}$) and FEL ($^P[E_x]_{FEL}$) are predefined and are in accordance with equation 1 above.

The concentration of the at least one adjustable excipient in the FEL ($^P[E_x]_{FEL}$) is predefined in accordance with the predefined concentrations of the at least one adjustable excipient in the VEL/LDS and the target concentration of the at least one adjustable excipient in the LPC ($^T[E_x]_{LPC}$). Once the concentrations have been determined for the particular embodiment, they are constant and do not change from batch to batch.

The target volume of the LPC ($^{T}V_{LPC}$) is chosen according to the appropriate target volume for the desired LPC. It may be for example chosen based on the maximum volume of a vessel used in the batch mixing process. Furthermore, the predefined volume of the FEL ($^{P}V_{FEL}$) is chosen in accordance with the chosen target volume of the LPC. In one embodiment, $^{P}V_{FEL}$ is smaller than $^{T}V_{LPC}$. In a preferred embodiment, $^{P}V_{FEL}$ is at least 10% of $^{T}V_{LPC}$.

In a preferred embodiment, the target volume of the LPC according to the invention can also be expressed as:

$$^{T}V_{LPC} = {}^{P}V_{FEL} + {}^{v}V_{VEL} + {}^{v}V_{LDS(total)}, \text{ wherein } {}^{V}V_{LDS(total)} = \sum_{i=1}^{\infty} V_{LDS(i)}.$$

Accordingly, the volume of the sum of $^{v}V_{VEL} + {}^{v}V_{LDS(total)}$ is also predefined. Furthermore, the predefined volume of the sum of $^{v}V_{VEL} + {}^{v}V_{LDS(total)}$ is chosen in accordance with the chosen target volume of the LPC. In one embodiment, the sum of $^{v}V_{VEL} + {}^{v}V_{LDS(total)}$ is smaller than $^{T}V_{LPC}$. In a preferred embodiment, the sum of $^{v}V_{VEL} + {}^{v}V_{LDS(total)}$ is up to 90% of $^{T}V_{LPC}$.

Hence, the variable volume parameters $^{v}V_{LDS(total)}$ and $^{v}V_{VEL}$ are calculated for each batch of LPC according to the variable concentrations of biological active ingredient in each of the at least one LDS. It is preferred that all other parameters are predefined according to the desired target values of the LPC.

Thus, one preferred embodiment of the method of the invention provides a batch mixing process for preparing a liquid pharmaceutical composition (LPC) wherein only the variable volume of the $^{v}V_{LDS(total)}$ and $^{v}V_{VEL}$ that need to be added to each batch of LPC need to be calculated. Hence, the method provides an efficient and streamlined process that is applicable to any LPC of choice and can be used for the large-scale manufacture of liquid pharmaceutical compositions, such as tetravalent dengue vaccines, in particular live attenuated tetravalent dengue vaccines. In a preferred embodiment, the tetravalent dengue vaccine includes all four live, attenuated dengue serotypes including dengue serotype 1 such as dengue 2/1 chimera or TDV-1 represented by SEQ ID NO: 1 and/or SEQ ID NO:2 , dengue serotype 2 or TDV-2 represented by SEQ ID NO: 3 and/or SEQ ID NO:4, dengue serotype 3 such as dengue serotype 2/3 or TDV-3 represented by SEQ ID NO: 5 and/or SEQ ID NO:6, dengue serotype 4 such as dengue serotype 2/4 or TDV-4 represented by SEQ ID NO: 7 and/or SEQ ID NO:8.

The present invention further relates to a product or pharmaceutical composition obtainable or obtained by the process described above and the use of said product or pharmaceutical composition in a method of treating or preventing a disease, in particular in a method of preventing dengue disease.

**DEFINITIONS**

Unless defined otherwise, all technical and scientific terms used herein have the same meaning as commonly understood by one of ordinary skill in the art to which the invention pertains. Although, any processes and materials similar or equivalent to those described herein can be used in practice for testing of the present invention, the preferred materials and processes are described herein. In describing and claiming the present invention, the following terminology will be used in accordance with the definitions set out below.

Throughout the description, references to the words "can" and "may", (e.g. such as the phrase "can be" or "may be") are not construed as limiting the invention to specific embodiments but rather are illustrative and non-limiting examples.

Unless clearly indicated otherwise, use of the terms "a," "an," and the like refers to one or more.

The term "large-scale" also called "production scale" or "manufacturing scale" as used herein refers to isolation of the virus achievable from volumes of starting sample in the order of thousands of litres.

The term "liquid drug substance" or "LDS" refers to a liquid comprising a variable concentration of biological active agent and a predefined concentration of excipients. The predefined concentration of excipients is set by the process of preparing the LDS.

The term "predefined concentration" refers to a given concentration of an excipient in a given liquid, which is set in advance and therefore, always remains the same from batch to batch. The term "predefined volume" refers to a given volume of liquid which is set in advance and therefore always remains the same from batch to batch. The term "predefined" refers to values that are set by circumstances outside the mixing process, i.e. the concentration of excipients in the LDS, and also refers to values set for the mixing process, i.e. the concentration of excipients in the FEL and the volume of FEL and the combined volume of LDS and VEL.

The term "variable volume" refers to a volume of VEL or LDS, which can vary from batch to batch depending on the concentration of the biological active agent(s) in the LDS(s). The term "variable concentration" refers to a concentration of biological active agent in the LDS, which can vary from batch to batch.

The term "target concentration" refers to the desired end concentrations of excipients and active agents set in advance in the liquid pharmaceutical composition (LPC) obtained/obtainable by the process according to the present invention. The term "target volume" refers to the desired end volume set in advance of the liquid pharmaceutical composition (LPC) obtained/obtainable by the process according to the present invention.

As used herein, the term "excipient" refers to a substance added to a liquid pharmaceutical composition in addition to the biological active agent. This can include substances used for the purpose of enhancing stabilization of the active agent, salts, carbohydrates (such as e.g., sugars), surfactants, proteins, bulking agents, fillers, or agents that in combination with the active agent can confer a therapeutic enhancement of the composition. In particular, excipients may refer to salts, carbohydrates, non-ionic surfactants and albumins. Excipients may also refer to buffers such as e.g., phosphate buffer. Excipients may also refer to buffers such as e.g. phosphate buffer. Phosphate buffer is obtainable by admixing potassium buffer salts such as e.g. potassium dihydrogen phosphate and disodium hydrogen phosphate, in a set ratio. Within the meaning of this invention phosphate buffer is considered as a single excipient. The excipients according to the present invention may be described by referring to the admixed (dry) excipients.

The term "non-ionic surfactant" means a surfactant that contains neither positively nor negatively charged functional groups. In contrast to anionic and cationic surfactants, non-ionic surfactants do not ionize in solution. Non-ionic surfactants may be selected from block copolymers, sorbitan esters, ethoxylated or propoxylated sorbitan esters, alkyl-polyglycosides (APG), alkoxylated mono- or di-alkylamines, fatty acid monoethanolamides (FAMA), fatty acid diethanolamides (FADA), ethoxylated fatty acid monoethanolamides (EFAM), propoxylated fatty acid monoethanolamides (PFAM), polyhydroxy alkyl fatty acid amides, or N-acyl N-alkyl derivatives of glucosamine (glucamides, GA, or fatty acid glucamide, FAGA), and combinations thereof.

The term "albumin" refers to the albumin family of globular non-glycosylated proteins. They are water-soluble, moderately soluble in concentrated salt solutions, and experience heat denaturation. Suitable albumins for use in the process of the present invention include mammalian serum albumins such as human serum albumin and bovine serum albumin or lactalbumin. Serum albumin is one of the most common proteins in vertebrate blood and has multiple functions. The protein is 585 amino acids with a molecular weight of 66,500 D. Human serum albumin is not glycosylated and has a single free thiol group. The human serum albumin may be recombinant human serum albumin, or it may be human serum albumin purified from human serum. Preferably, human serum albumin purified from human serum is used.

Within the meaning of this invention, the term "set excipient" refers to an excipient which has the same concentration in the VEL, LDS, and FEL and consequently, also in the LPC. The concentration does not have to be adjusted to obtain a target concentration in the LPC, instead the concentration is already the target concentration from the outset.

Within the meaning of this invention, the term "adjustable excipient" refers to an excipient, which does not have the target concentration of the LPC in the LDS, but instead needs to be adjusted to become the target concentration in LPC by appropriate mixing with VEL and FEL.

As used herein, the term "biological active agent" refers to a compound which is biologically active and is associated with a particular pharmacological effect. A biological active agent is generally a substance which is manufactured in, extracted from, or semi-synthesized from biological sources. Biological active agents may be isolated from living sources, such as human, animal, plant, fungal, or microbial sources. In particular, biological active agents are often derived from tissue cultures of human or animal cell lines (e.g. Vero cell lines). In a particular embodiment, biological active agents refer to viruses, such as e.g. dengue viruses. Different biological active agents may, therefore, refer to different virus or to different serotypes of the same virus.

The process of the present invention can be used for preparing a liquid pharmaceutical composition (LPC) comprising flaviviruses. The genus flavivirus comprises enveloped positive-stranded RNA viruses such as West Nile (WN) virus, Japanese Encephalitis virus (JEV), Zika virus, Dengue fever virus, yellow fever virus (YF), Kyasanur Forest disease virus, Murray Valley encephalitis virus, St. Louis encephalitis virus, Tick-borne encephalitis virus, West Nile encephalitis virus, Central European encephalitis (TBE-W) virus, Far Eastern encephalitis (TBE-FE) virus, Kunjin virus, Tyuleniy virus, Ntaya virus, Uganda S virus, Modoc virus, BVDV (e.g, strains NADL, and 890), CSFV Alfort 187, BDV BD31 virus, and/or GB virus-A, -B, and/or -C.

The process of the present invention can be used for preparing a liquid pharmaceutical composition (LPC) comprising dengue viruses, i.e., each of the dengue serotypes. Dengue virus as used herein is a single stranded, positive sense RNA virus of the family flaviviridae. The family flaviviridae includes three genera, flavivirus, hepacivirus and pestivirus. As used herein, the term "dengue serotype" refers to a species of dengue virus which is defined by its cell surface antigens and therefore can be distinguished from other dengue serotypes by serological methods known in the art. At present, four serotypes of dengue virus are known, i.e., dengue serotype 1 (DENV-1), dengue serotype 2 (DENV-2), dengue serotype 3 (DENV-3) and dengue serotype 4 (DENV-4). According to this definition, a dengue serotype 1 can be, for example, the chimeric dengue serotype 2/1 strain (TDV-1) described herein. Thus, the process of the present invention can also be used for large-scale production and manufacture of four live, attenuated dengue virus strains separately.

As used herein, the term "live, attenuated dengue virus" refers to a viable and infectious dengue virus which is mutated to provide reduced virulence. The live, attenuated dengue virus can be a dengue virus in which all components are derived from the same dengue serotype or it can be a chimeric dengue virus having parts from two or more dengue serotypes. A "virus strain" and in particular a "dengue virus strain" is a genetic subtype of a virus, in particular of a dengue virus, which is characterized by a specific nucleic acid sequence. A dengue serotype may comprise different strains with different nucleic acid sequences which have the same cell surface antigens and are therefore recognized by the same antibodies. A dengue virus strain can be a dengue virus in which all components are derived from the same dengue serotype or it can be a chimeric dengue virus having parts from two or more dengue serotypes.

As used herein "dengue-1", "dengue-2", "dengue-3" and "dengue-4" may each refer to a chimeric or non-chimeric live attenuated dengue serotype.

A "chimeric virus" or "chimeric strain" or "chimeric virus strain" in general comprises parts from at least two different viruses. For example, a chimeric virus can comprise the prM and E proteins of dengue virus and the other proteins from another flavivirus. The chimeric virus can comprise the prM and E proteins of dengue virus and the other proteins from another flavivirus such as yellow fever virus, Zika virus, West Nile virus, Japanese encephalitis virus, St. Louis encephalitis virus and tick-borne encephalitis virus. The chimeric virus can comprise the prM and E proteins of dengue virus and the other proteins from yellow fever virus strain YF-17D. Such chimeric viruses are present in the commercial product Dengvaxia® and are described in, e.g., WO 98/37911, WO 03/101397, WO 2007/021672, WO 2008/007021, WO 2008/047023 and WO 2008/065315.

A "chimeric dengue virus" or "chimeric dengue serotype strain" or "chimeric dengue strain" preferably comprises parts from at least two different dengue serotypes, i.e. a dengue-dengue chimera. Such chimeric dengue viruses are described in WO 01/060847 A2, WO 2014/150939 A2 and WO 2017/179017 A1.

Certain embodiments of the present invention are thus including the manufacture of tetravalent compositions of 4 chimeric dengue strains, and/or tetravalent compositions of 4 attenuated dengue strains, such as e.g. 4 attenuated chimeric dengue strains or the "TAK-003" product (see below). A chimeric dengue virus may include parts from a different flavivirus such as yellow fever virus, Zika virus, West Nile virus, Japanese encephalitis virus, St. Louis encephalitis virus and tick-borne encephalitis virus. For example, the chimeric dengue virus described herein can include parts from the yellow fever virus.

As used herein, a "chimeric dengue serotype 2/1 strain" or "DENV-2/1 chimera" or "TDV-1" refers to a dengue virus chimeric construct which comprises parts from both DENV-2 and DENV-1. Preferably, in the chimeric dengue serotype 2/1 strain the prM and E proteins from DENV-1 replace the prM and E proteins from DENV-2.

As used herein, "TDV-2" refers to a molecularly characterized and cloned dengue serotype 2 strain derived from the live attenuated DEN-2 PDK-53 virus strain. The PDK-53 strain is described for example in Bhamarapravati et al. (1987) Bulletin of the World Health Organization 65(2): 189-195. In one embodiment, the TDV-2 strain served as a backbone for the chimeric TDV-1, TDV-3 and TDV-4 strains into which parts from the TDV-1, TDV-3 and TDV-4 strains were introduced. TDV-1, TDV-2, TDV-3 and TDV-4 together form a tetravalent dengue virus composition, "TAK-003", a dengue vaccine candidate for which regulatory filings have recently been submitted by Takeda.

As used herein, a "chimeric dengue serotype 2/3 strain" or "DENV-2/3 chimera" or "TDV-3" refers to a dengue virus chimeric construct which comprises parts from both DENV-2 and DENV-3. Preferably, in the chimeric dengue serotype 2/3 strain the prM and E proteins from DENV-3 replace the prM and E proteins from DENV-2.

As used herein, a "chimeric dengue serotype 2/4 strain" or "DENV-2/4 chimera" or "TDV-4" refers to a dengue virus chimeric construct which comprises parts from both DENV-2 and DENV-4. Preferably, in the chimeric dengue serotype 2/4 strain the prM and E proteins from DENV-4 replace the prM and E proteins from DENV-2.

As used herein, "TDV" refers to a tetravalent live attenuated dengue vaccine that comprises a mixture of the four live attenuated dengue virus strains TDV-1, TDV-2, TDV-3 and TDV-4 expressing surface antigens from the four dengue serotypes DENV-1, DENV-2, DENV-3 and DENV-4, respectively. In one embodiment (e.g. also in the examples), TDV-1 is characterized by the nucleotide sequence according to SEQ ID No. 1 and/or the amino acid sequence according to SEQ ID No. 2. In one embodiment, TDV-2 is characterized by the nucleotide sequence according to SEQ ID No. 3 and/or the amino acid sequence according to SEQ ID No. 4. In one embodiment, TDV-3 is characterized by the nucleotide sequence according to SEQ ID No. 5 and/or the amino acid sequence according to SEQ ID No. 6. In one embodiment, TDV-4 is characterized by the nucleotide sequence according to SEQ ID No. 7 and/or the amino acid sequence according to SEQ ID No. 8.

As used herein, the terms "subject" or "subjects" generally refer to human subjects (e.g. infants, children or adults).

As used herein, the term "dengue disease" refers to the disease, which is caused by infection with dengue virus. Symptoms of dengue disease include sudden high fever, headaches, joint and muscle pain, nausea, vomiting and skin rashes. The term dengue disease also includes the more severe forms of dengue haemorrhagic fever (DHF) and dengue shock syndrome (DSS). Symptoms of DHF include increased vascular permeability, hypovolemia and abnormal blood clotting mechanisms. Subjects with DHF may present with severe manifestations of plasma leakage and haemorrhage. When a subject with DHF experiences shock he or she will be categorized as having DSS. Symptoms of DSS include bleeding that may appear as tiny spots of blood on the skin and larger patches of blood under the skin. Prolonged shock is the main factor associated with complications including massive gastrointestinal haemorrhage that can lead to death.

As used herein, "preventing" refers to preventing a subject from developing one or more symptoms of a disease; in particular dengue disease because of an infection with a dengue virus. In a particular embodiment, preventing dengue disease includes preventing DHS and/or DSS.

"Dengue virus vaccine" as used herein means either a monovalent vaccine, i.e., one of the four known dengue serotypes, a divalent vaccine, i.e., two of the four serotypes, a trivalent vaccine, i.e., three of the four serotypes, or tetravalent vaccine, i.e., all four serotypes.

The term "viral titer", "virus titer" or "titer" as used herein refers to the concentration of infectious viral particles. The viral titer can be determined using an assay which measures the ability of a flaviviral particle to bind to, penetrate and deliver its genome to the cytoplasm of a host cell, thereby allowing expression of structural proteins in a host cell. Viral titer in the context of the invention can be measured using a immunofluorescence assay. Alternatively, any other analytical method can be used to measure the viral titer, for example, viral titer can be measured by a functional assay, such as an assay described in Xiao et al., Exp. Neurobiol. 144:113-124, 1997, or Fisher et al., J. Virol. 70:520-532, 1996. The virus titer is herein expressed in $\log_{10}$ PFU/ml. Virus concentrations are determined by an immune focus assay that results in plaque forming units (PFU). In one example, the immune focus assay is carried out as described in detail in section 2.5 of Brewoo et al. (Vaccine. 2012 February 14; 30(8): 1513-1520. doi:10.1016/j.vaccine.2011.11.072.)."

The term "dry" (or "dried product") refers to a solid material with a residual water content of less than about 10% w/w. Dried products are preferably dried to residual liquid (water) contents of 8% or less, 5% or less, or preferably from about 0.1 % to about 5 %. Preferably, the residual water content is determined in accordance with the Karl Fischer titration method.

The term "lyophilization" as used herein refers to freeze-drying of an aqueous solution, resulting in a powder or solid. Lyophilization involves manipulating the temperature and pressure of the solution so that the phase of the solution can move directly from the frozen state to the gaseous state without moving through the liquid phase/state. This is achieved by cooling the solution and lowering the pressure to below the triple point of water (the temperature and pressure at which water can exist in equilibrium in the liquid, solid, and gaseous states - which is 0.01°C). This allows for the removal of the solvent from the product without subjecting the product to intense heat.

SUMMARY OF THE ABBREVIATIONS

| Abbreviation | Meaning | Abbreviations for 4 different adjustable excipients (a, b, c and d) | Abbreviations for 4 different biological active agents (1, 2, 3 and 4) in 4 different LDSs (1, 2, 3 and 4) | For the dengue serotypes dengue-1 ($D_1$), dengue-2 ($D_2$), dengue-3 ($D_3$), dengue-4 ($D_4$) |
|---|---|---|---|---|
| $^T[A_i]_{LPC}$ | Target concentration of the at least one biological active agent in the LPC | - | $^T[A_1]_{LPC}$, $^T[A_2]_{LPC}$, $^T[A_3]_{LPC}$, $^T[A_4]_{LPC}$ | $^T[D_1]_{LPC}$, $^T[D_2]_{LPC}$, $^T[D_3]_{LPC}$, $^T[D_4]_{LPC}$ |
| $^T[E_x]_{LPC}$ | Target concentration of the at least one adjustable excipient in the LPC | $^T[E_a]_{LPC}$, $^T[Et_b]_{LPC}$, $^T[E_c]_{LPC}$, $^T[E_d]_{LPC}$ | - | - |
| $^TV_{LPC}$ | Target volume of the LPC | - | - | - |
| $^V[A_i]_{LDS}$ | Variable concentration of the at least one biological active agent in the LDS | - | $^V[A_1]_{LDS}$, $^V[A_2]_{LDS}$, $^V[A_3]_{LDS}$, $^V[A_4]LDS$ or $^V[A_1]_{LDS1}$, $^V[A_2]_{LDS2}$, $^V[A_3]_{LDS3}$, $^V[A_4]_{LDS4}$ | $^V[D_1]_{LDS}$, $^V[D_2]_{LDS}$, $^V[D_3]_{LDS}$, $^V[D_4]_{LDS}$ |
| $^P[E_x]_{LDS}$ | Predefined concentration of the at least one adjustable excipient in the LDS | $^P[E_a]_{LDS}$, $^P[E_b]_{LDS}$, $^P[E_c]_{LDS}$, $^P[E_d]_{LDS}$ | - | - |
| $^VV_{LDS(i)}$ | Variable volume of the LDS | - | $^VV_{LDS(1)}$, $^VV_{LDS(2)}$, $^VV_{LDS(3)}$, $^VV_{LDS(4)}$ | - |

(continued)

| Abbreviation | Meaning | Abbreviations for 4 different adjustable excipients (a, b, c and d) | Abbreviations for 4 different biological active agents (1, 2, 3 and 4) in 4 different LDSs (1, 2, 3 and 4) | For the dengue serotypes dengue-1 ($D_1$), dengue-2 ($D_2$), dengue-3 ($D_3$), dengue-4 ($D_4$) |
|---|---|---|---|---|
| $^VV_{LDS(total)}$ | Combined total volume of all LDSs | - | - | - |
| $^P[E_x]_{VEL}$ | Predefined concentration of the at least one adjustable excipient in the VEL | $^P[E_a]_{VEL}, ^P[E_b]_{VEL}, ^P[E_c]_{VEL}, ^P[E_d]_{VEL}$ | - | - |
| $^vV_{VEL}$ | Variable volume of VEL | - | - | - |
| $^pV_{(VEL+LDS)}$ | Predefined combined total volume of all LDSs and the VEL | - | - | - |
| $^P[E_x]_{FEL}$ | Predefined concentration of the at least one adjustable excipient in the FEL | $^P[E_a]_{FEL}, ^P[E_b]_{FEL}, ^P[E_c]_{FEL}, ^P[E_d]_{FEL}$ | - | - |
| $^pV_{FEL}$ | Predefined volume of FEL | - | - | - |
| $^vV_{IL}$ | Variable volume of the IL | | | |

## BRIEF DESCRIPTION OF THE DRAWINGS

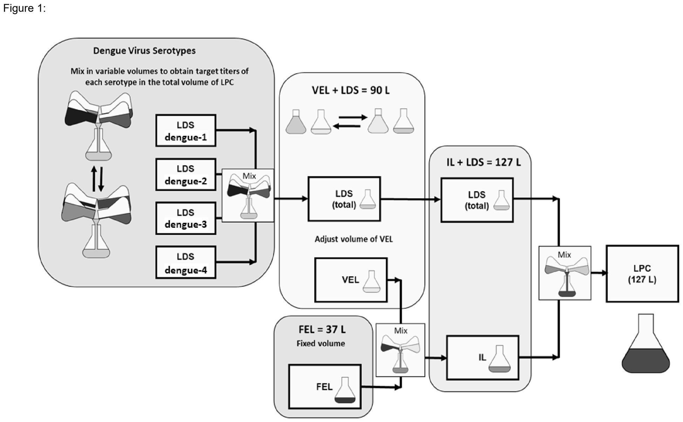

Figure 1: A schematic drawing illustrating the mixing process according to Example 1 of the present application.

## DETAILED DESCRIPTION

The invention provides a batch mixing process for preparing a liquid pharmaceutical composition (LPC) with a predefined, consistent target concentration of at least one biological active agent. The at least one biological active agent is prepared through a biological process, e.g. cell culture processes such as protein expression, the end product of which is a liquid drug substance (LDS) with a batch-to-batch variable concentration of the at least one biological active agent. In order to prepare the LPC with the correct concentration of the at least one biological active agent, different volumes of each of the at least one LDS(s) must be used. However, the LPC additionally comprises at least one adjustable excipient. Therefore, the at least one biological active agent and the at least one adjustable excipient are variables that need to be adjusted, resulting in the need to calculate concentrations and volumes for each variable. Especially when the LPC comprises more than one, for example four, biological active agents and more than one, for example four, adjustable excipients, the process of mixing solutions correctly would be complicated and inefficient. Hence, the invention provides an efficient batch mixing process. Using this process, only the volume of each of the at least one LDS(s) and the volume of the variable excipient liquid (VEL) need to be adjusted.

The invention comprises the steps of providing a variable excipient liquid (VEL) comprising the at least one adjustable excipient at a predefined concentration ($^P[E_x]_{VEL}$), one or more liquid drug substance (LDS) comprising one biological active agent at a variable concentration ($^V[A_i]_{LDS}$) and the at least one adjustable excipient at a predefined concentration ($^P[E_x]_{LDS}$), and a fixed excipient liquid (FEL) comprising the at least one adjustable excipient at a predefined concentration ($^P[E_x]_{FEL}$). The VEL and FEL can be provided as stock solutions, since the concentration of the at least one adjustable excipients therein does not change from batch to batch, i.e. when different batches of LPC are prepared. Thus, the same VEL and FEL can be used to prepare each batch of LPC, wherein each batch of LPC may comprise different batches of LDS(s).

Hence, in one embodiment, the VEL and FEL concentration of the at least one adjustable excipient do not change when different batches of LPC are prepared. This means that the same stock preparations of VEL and FEL can be used to prepare each batch of LPC.

It is a feature of the present invention that the LDS(s) and the FEL have the same predefined concentration of the at least one adjustable excipient, while the FEL has a different predefined concentration of the same at least one adjustable excipient, according to the formula $^P[E_x]_{VEL} = {}^P[E_x]_{LDS} \neq {}^P[E_x]_{FEL}$, This means that in order to arrive at an LPC comprising the same at least one adjustable excipient at a predefined concentration of choice, only the volume of VEL needs to be adjusted. The variable volume of the at least one LDS is calculated according to the concentration of the biological active agent, which is variable due to its nature. Hence, the VEL is adjusted based on the volume of LDS(s) needed to provide the target concentration of the at least one biological active ingredient in the LPC. The fixed volume of the FEL and the fixed volume of the sum of (LDS(s) + VEL) are predefined and correspond to the predefined volume and target concentration of the at least one adjustable excipient of the LPC. The FEL, LDS(s), VEL and LPC may also comprise at least one set excipient at a fixed concentration.

### Excipients

In one embodiment, the VEL comprises at least one, at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen or at least twenty adjustable excipients at a predefined concentration. In another embodiment, the VEL comprises between one and twenty, between one and fifteen, between one and ten or between one and eight adjustable excipients at a predefined concentration. In a preferred embodiment, the VEL comprises at least four adjustable excipients at a predefined concentration.

In one embodiment, the FEL comprises at least one, at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen or at least twenty adjustable excipients at a predefined concentration. In another embodiment, the FEL comprises between one and twenty, between one and fifteen, between one and ten or between one and eight adjustable excipients at a predefined concentration. In a preferred embodiment, the FEL comprises at least four adjustable excipients at a predefined concentration.

In one embodiment, the LDS comprises at least one, at least two, at least three, at least four, at least five, at least six, at least seven, at least eight, at least nine, at least ten, at least eleven, at least twelve, at least thirteen, at least fourteen, at least fifteen, at least sixteen, at least seventeen, at least eighteen, at least nineteen or at least twenty adjustable excipients at a predefined concentration. In another embodiment, the LDS comprises between one and twenty, between one and fifteen, between one and ten or between one and eight adjustable excipients at a predefined concentration. In a preferred embodiment, the LDS comprises at least four adjustable excipients at a predefined concentration.

In one embodiment, the VEL, LDS, or FEL comprises at least one excipient selected from salts, sugars, non-ionic surfactants, and proteins. In one embodiment, the VEL, LDS, and FEL each comprise at least one excipient selected from salts, sugars, non-ionic surfactants, and proteins.

In one embodiment, the VEL and LDS each comprise the same at least one excipient. In one embodiment, the VEL and LDS each comprise the same at least one excipient at the same predefined concentrations.

In another embodiment, the VEL and LDS each comprise the same at least one excipient at different predefined concentrations.

In one embodiment, the FEL, VEL and LDS each comprise the same at least one excipient. In one embodiment, the VEL and LDS each comprise the same at least one excipient at the same predefined concentrations and the FEL comprises the same at least one excipient at a different predefined concentration.

In another embodiment, each batch of FEL, VEL and LDS comprise the same at least one excipient. In another embodiment, each batch of FEL, VEL and LDS comprise the same at least one adjustable excipient and at least one set excipient.

In one embodiment, each batch of the liquid pharmaceutical composition (LPC) comprises the same predefined target concentration of at least one excipient. In one embodiment, each batch of the liquid pharmaceutical composition (LPC) comprises the same predefined target concentration of at least one adjustable excipient and at least one set excipient.

### 1. Adjustable excipients: salts

The salts can be anhydrous or hydrates. The salts can be either one, two, three, or four sodium salts, or one, two, three, or four potassium salts, or one, two, three, or four magnesium salts. Each possibility can be combined with the other possibility. Thus, each possibility represents a separate embodiment of the invention.

For example, the sodium salt can be sodium fluoride, or sodium chloride, or sodium bromide, or sodium iodide, or sodium sulphate, or disodium salts such as disodium phosphate, or sodium dihydrogen phosphate or disodium hydrogen phosphate or disodium hydrogen phosphate, dihydrate, or sodium bicarbonate or sodium carbonate. For example, the potassium salt can be potassium fluoride, or potassium chloride, or potassium iodide, or potassium sulphate, or potassium dihydrogen phosphate or potassium dihydrogen sulphate or potassium bicarbonate or potassium carbonate. For example, the magnesium salt can be magnesium fluoride, or magnesium chloride, or magnesium iodide, or magnesium sulphate, or magnesium dihydrogen phosphate or magnesium bicarbonate or magnesium carbonate. In one embodiment, the at least one salt is a sodium salt, preferably selected from sodium chloride and disodium hydrogen phosphate dihydrate. In another embodiment, the at least one salt is a potassium salt, preferably selected from potassium dihydrogen phosphate and potassium chloride.

In one embodiment, the VEL, LDS or FEL comprises at least one sodium salt and at least one potassium salt.

In one embodiment, the VEL, LDS and FEL each comprise at least one sodium salt and at least one potassium salt.

In one embodiment, the VEL, LDS or FEL comprises sodium chloride and disodium hydrogen phosphate dihydrate.

In one embodiment, the VEL, LDS and FEL each comprise sodium chloride and disodium hydrogen phosphate dihydrate.

In one embodiment, the VEL, LDS or FEL comprises potassium dihydrogen phosphate and potassium chloride.

In one embodiment, the VEL, LDS and FEL each comprise potassium dihydrogen phosphate and potassium chloride.

In one embodiment, the VEL, LDS or FEL comprises sodium chloride, disodium hydrogen phosphate dihydrate, potassium dihydrogen phosphate and potassium chloride.

In one embodiment, the VEL, LDS and FEL each comprise sodium chloride, disodium hydrogen phosphate dihydrate, potassium dihydrogen phosphate and potassium chloride.

Each of the above salts can preferably be used within the range of 0.9 mM to 130 mM in the VEL, LDS, or FEL.

For example, the VEL can comprise sodium chloride in the range 100 to 130 mM, 110 to 130 mM, 120 to 130 mM and other three salts within the range of 0.9 to 6 mM. For example, the VEL can comprise potassium chloride in the range 100 to 130 mM, 110 to 130 mM, 120 to 130 mM and other three salts within the range of 0.9 to 6 mM. For example, VEL can comprise magnesium chloride in the range 100 to 130 mM, 110 to 130 mM, 120 to 130 mM and other three salts within the range of 0.9 to 6 mM.

For example, the VEL can comprise sodium fluoride in the range 100 to 130 mM, 110 to 130 mM, 120 to 130 mM and other three salts within the range of 0.9 to 6 mM. For example, the VEL can comprise potassium fluoride in the range 100 to 130 mM, 110 to 130 mM, 120 to 130 mM and other three salts within the range of 0.9 to 6 mM.

For example, the VEL can comprise sodium carbonate in the range 100 to 130 mM, 110 to 130 mM, 120 to 130 mM and other three salts within the range of 0.9 to 6 mM. For example, the VEL can comprise potassium carbonate in the range 100 to 130 mM, 110 to 130 mM, 120 to 130 mM and other three salts within the range of 0.9 to 6 mM.

For example, the VEL can comprise sodium chloride in the range 100 to 130 mM, 110 to 130 mM, 120 to 130 mM, and potassium chloride in the range 1 mM to 2 mM, 1 mM to 1.5 mM and two other salts within the range of 0.9 to 6 mM. For example, the VEL can comprise sodium chloride in the range 100 to 130 mM, 110 to 130 mM, 120 to 130 mM, and magnesium chloride in the range 1 mM to 2 mM, 1 mM to 1.5 mM and two other salts within the range of 0.9 to 6 mM. For example, the VEL can comprise potassium chloride in the range 100 to 130 mM, 110 to 130 mM, 120 to 130 mM, and magnesium chloride in the range 1 mM to 2 mM, 1 mM to 1.5 mM and two other salts within the range of 0.9 to 6 mM.

For example, the VEL can comprise sodium fluoride in the range 100 to 130 mM, 110 to 130 mM, 120 to 130 mM, and potassium chloride in the range 1 mM to 2 mM, 1 mM to 1.5 mM and two other salts within the range of 0.9 to 6 mM. For example, the VEL can comprise sodium fluoride in the range 100 to 130 mM, 110 to 130 mM, 120 to 130 mM, and potassium carbonate in the range 1 mM to 2 mM, 1 mM to 1.5 mM and two other salts within the range of 0.9 to 6 mM. For example, the VEL can comprise sodium fluoride in the range 100 to 130 mM, 110 to 130 mM, 120 to 130 mM, and magnesium chloride in the range 1 mM to 2 mM, 1 mM to 1.5 mM and two other salts within the range of 0.9 to 6 mM. For example, the VEL can comprise sodium iodide in the range 100 to 130 mM, 110 to 130 mM, 120 to 130 mM, and potassium carbonate in the range 1 mM to 2 mM, 1 mM to 1.5 mM and two other salts within the range of 0.9 to 6 mM.

For example, the VEL can comprise sodium chloride in the range 100 to 130 mM, 110 to 130 mM, 120 to 130 mM, and potassium chloride in the range 1 mM to 2 mM, 1 mM to 1.5 mM, potassium dihydrogen phosphate within the range 0.9 to 1.2 mM, 0.9 to 1.0 mM and one other salt within the range of 4 to 6 mM, 5 to 6 mM. For example, the VEL can comprise sodium chloride in the range 100 to 130 mM, 110 to 130 mM, 120 to 130 mM, and potassium phosphate in the range 1 mM to 2 mM, 1 mM to 1.5 mM, potassium dihydrogen sulphate within the range 0.9 to 1.2 mM, 0.9 to 1.0 mM and one other salt within the range of 4 to 6 mM, 5 to 6 mM. For example, the VEL can comprise sodium chloride in the range 100 to 130 mM, 110 to 130 mM, 120 to 130 mM, and potassium phosphate in the range 1 mM to 2 mM, 1 mM to 1.5 mM, sodium dihydrogen sulphate within the range 0.9 to 1.2 mM, 0.9 to 1.0 mM and one other salt within the range of 4 to 6 mM, 5 to 6 mM.

For example, the VEL can comprise sodium chloride in the range 100 to 130 mM, 110 to 130 mM, 120 to 130 mM, and potassium chloride in the range 1 mM to 2 mM, 1 mM to 1.5 mM, potassium dihydrogen phosphate within the range 0.9 to 1.2 mM, 0.9 to 1.0 mM and disodium hydrogen phosphate dihydrate within the range of 4 to 6 mM, 5 to 6 mM. For example, the VEL can comprise potassium chloride in the range 100 to 130 mM, 110 to 130 mM, 120 to 130 mM, and magnesium chloride in the range 1 mM to 2 mM, 1 mM to 1.5 mM, sodium dihydrogen phosphate within the range 0.9 to 1.2 mM, 0.9 to 1.0 mM and disodium hydrogen phosphate dihydrate within the range of 4 to 6 mM, 5 to 6 mM. For example, the VEL can comprise potassium chloride in the range 100 to 130 mM, 110 to 130 mM, 120 to 130 mM, and magnesium chloride in the range 1 mM to 2 mM, 1 mM to 1.5 mM, sodium dihydrogen phosphate within the range 0.9 to 1.2 mM, 0.9 to 1.0 mM and magnesium carbonate within the range of 4 to 6 mM, 5 to 6 mM.

For each of the above examples, the concentration of an adjustable excipient is the same in the VEL and the LDS Each of the above salts can be used within the range of 3 mM to 50 mM in the FEL.

For example, the FEL can comprise sodium chloride in the range 20 to 50 mM, 30 to 50 mM, 40 to 50 mM and other three salts within the range of 3 to 25 mM. For example, the FEL can comprise potassium chloride in the range 20 to 50 mM, 30 to 50 mM, 40 to 50 mM and other three salts within the range of 3 to 25 mM. For example, the FEL can comprise magnesium chloride in the range 20 to 50 mM, 30 to 50 mM, 40 to 50 mM and other three salts within the range of 3 to 25 mM.

For example, the FEL can comprise sodium fluoride in the range 20 to 50 mM, 30 to 50 mM, 40 to 50 mM and other three salts within the range of 3 to 25 mM. For example, the FEL can comprise potassium fluoride in the range 20 to 50 mM, 30 to 50 mM, 40 to 50 mM and other three salts within the range of 3 to 25 mM.

For example, the FEL can comprise sodium carbonate in the range of 20 to 50 mM, 30 to 50 mM, 40 to 50 mM and other three salts within the range of 3 to 25 mM. For example, the FEL can comprise potassium carbonate in the range 20 to 50 mM, 30 to 50 mM, 40 to 50 mM and other three salts within the range of 3 to 25 mM.

For example, the FEL can comprise sodium chloride in the range 20 to 50 mM, 30 to 50 mM, 40 to 50 mM, and potassium chloride in the range 4 to 6 mM, 5 to 6 mM and two other salts within the range of 3 to 25 mM. For example, the FEL can comprise sodium chloride in the range of 20 to 50 mM, 30 to 50 mM, 40 to 50 mM, and magnesium chloride in the range 4 to 6 mM, 5 to 6 mM and two other salts within the range of 3 to 25 mM. For example, the FEL can comprise potassium chloride in the range of 20 to 50 mM, 30 to 50 mM, 40 to 50 mM, and magnesium chloride in the range 4 to 6 mM, 5 to 6 mM and two other salts within the range of 3 to 25 mM.

For example, the FEL can comprise sodium fluoride in the range 20 to 50 mM, 30 to 50 mM, 40 to 50 mM, and potassium chloride in the range 4 to 6 mM, 5 to 6 mM and two other salts within the range of 3 to 25 mM. For example, the FEL can comprise sodium fluoride in the range 20 to 50 mM, 30 to 50 mM, 40 to 50 mM, and potassium carbonate in the range 4 to 6 mM, 5 to 6 mM and two other salts within the range of 3 to 25 mM. For example, the FEL can comprise sodium fluoride in the range 20 to 50 mM, 30 to 50 mM, 40 to 50 mM, and magnesium chloride in the range 4 to 6 mM, 5 to 6 mM and two other salts within the range of 3 to 25 mM. For example, the FEL can comprise sodium iodide in the range 20 to 50 mM, 30 to 50 mM, 40 to 50 mM, and potassium carbonate in the range 4 to 6 mM, 5 to 6 mM and two other salts within the range of 3 to 25 mM.

For example, the FEL can comprise sodium chloride in the range of 20 to 50 mM, 30 to 50 mM, 40 to 50 mM, and potassium chloride in the range 4 to 6 mM, 5 to 6 mM, potassium dihydrogen phosphate within the range 3 to 4 mM, 3.2 to 4 mM and one other salt within the range of 18 to 22 mM. For example, the FEL can comprise sodium chloride in the range of 20 to 50 mM, 30 to 50 mM, 40 to 50 mM, and potassium phosphate in the range 4 to 6 mM, 5 to 6 mM, potassium dihydrogen sulphate within the range 3 to 4 mM, 3.2 to 4 mM and one other salt within the range of 18 to 22 mM. For example, the FEL can comprise sodium chloride in the range of 20 to 50 mM, 30 to 50 mM, 40 to 50 mM, and potassium phosphate in the range 4 to 6 mM, 5 to 6 mM, sodium dihydrogen sulphate within the range 3 to 4 mM, 3.2 to 4 mM and one other salt within the range of 18 to 22 mM.

For example, the FEL can comprise sodium chloride in the range 20 to 50 mM, 30 to 50 mM, 40 to 50 mM, and potassium chloride in the range 4 to 6 mM, 5 to 6 mM, potassium dihydrogen phosphate within the range 3 to 4 mM, 3.2 to 4 mM and disodium hydrogen phosphate dihydrate within the range of 18 to 22 mM, 19 to 22 mM, 20 to 22 mM. For example, the FEL can comprise potassium chloride in the range 20 to 50 mM, 30 to 50 mM, 40 to 50 mM, and magnesium chloride in the range 4 to 6 mM, 5 to 6 mM, sodium dihydrogen phosphate within the range 3 to 4 mM, 3.2 to 4 mM and disodium hydrogen phosphate dihydrate within the range of 18 to 22 mM, 19 to 22 mM, 20 to 22 mM. For example, the FEL can comprise potassium chloride in the range 20 to 50 mM, 30 to 50 mM, 40 to 50 mM, and magnesium chloride in the range 4 to 6 mM, 5 to 6 mM, sodium dihydrogen phosphate within the range 3 to 4 mM, 3.2 to 4 mM and magnesium carbonate within the range of 18 to 22 mM, 19 to 22 mM, 20 to 22 mM.

In one embodiment, the VEL and LDS each comprise the same at least one salt. In one embodiment, the VEL and LDS each comprise the same at least one salt at the same predefined concentrations.

In one embodiment, the FEL, VEL and LDS each comprise the same at least one salt. In one embodiment, the VEL and LDS each comprise the same at least one salt at the same predefined concentrations and the FEL comprises the same at least one salt at a different predefined concentration.

In one embodiment, one of the excipients comprised in the FEL, VEL and LDS is a phosphate buffer. The phosphate buffer comprises potassium dihydrogen phosphate and disodium hydrogen phosphate. In one embodiment, the phosphate buffer comprises potassium dihydrogen phosphate within the range 0.9 to 1.2 mM and disodium hydrogen phosphate dihydrate within the range of 4 to 6 mM.

In one embodiment, the total concentration of phosphate ions in the VEL and LDS is between 1 mM and 15 mM. In one embodiment, the total concentration of phosphate ions in the VEL and LDS is between 1 mM and 10 mM, between 1 mM and 9 mM, between 1 mM and 8 mM, between 1mM and 7mM, between 2 mM and 10 mM, between 2 mM and 9 mM, between 2 mM and 8 mM, between 2 mM and 7mM, between 3 mM and 10 mM, between 3 mM and 9 mM, between 3 mM and 8 mM, between 3 mM and 7mM, between 4 mM and 10 mM, between 4 mM and 9 mM, between 4 mM and 8 mM, between 4 mM and 7mM, between 5 mM and 10 mM, between 5 mM and 9 mM, between 5 mM and 8 mM, or between 5 mM and 7mM.

In one embodiment, the total concentration of phosphate ions in the FEL is between 10 mM and 50 mM. In one embodiment, the total concentration of phosphate ions in the FEL is between 10 mM and 45 mM, between 10 mM and 40 mM, between 10 mM and 35 mM, between 10 mM and 30 mM, between 15 mM and 45 mM, between 15 mM and 40 mM, between 15 mM and 35 mM, between 15 mM and 30 mM ,between 20 mM and 45 mM, between 20 mM and 40 mM, between 20 mM and 35 mM, or between 20 mM and 30 mM.

## 2. Adjustable excipients: sugars

The sugar in the FEL, VEL or LDS can be monosaccharides, (e.g. glucose, galactose, ribose, mannose, rhamnose, talose, xylose, or allose arabinose.), disaccharides (e.g. trehalose, sucrose, maltose, isomaltose, cellibiose, gentiobiose, laminaribose, xylobiose, mannobiose, lactose, or fructose), trisaccharides (e.g. acarbose, raffinose, melizitose, panose, or cellotriose) and sugar polymers (e.g. dextran, xanthan, pullulan, cyclodextrins, amylose, amylopectin, starch, celloologosaccharides, cellulose, maltooligosaccharides, glycogen, chitosan, or chitin). The sugar in the FEL, VEL or LDS can be sugar alcohols such as mannitol, sorbitol, arabitol, erythritol, maltitol, xylitol, glycitol, glycol, polyglycitol, polyethylene glycol, polypropylene glycol, and glycerol. The sugar in the FEL, VEL or LDS can be a non-reducing sugar. The sugar in the FEL, VEL or LDS can be sucrose, trehalose or its hydrates such as trehalose dihydrate.

Each of the above sugars can be used within the range of 120 to 160 g/L in the VEL or LDS and within the range of 180 to 220 g/L in the FEL. For example, sucrose can be within the range of 120 to 160 g/L, 130 to 160 g/L, 140 to 150 g/L in the VEL or LDS and 180 to 220 g/L, 190 to 210 g/L, 200 to 210 g/L in the FEL. For example, trehalose can be within the range of 120 to 160 g/L, 130 to 160 g/L, 140 to 150 g/L in the VEL or LDS and 180 to 220 g/L, 190 to 210 g/L, 200 to 210 g/L in the FEL. For example, trehalose dihydrate can be within the range of 120 to 160 g/L, 130 to 160 g/L, 140 to 150 g/L in the VEL and 180 to 220 g/L, 190 to 210 g/L, 200 to 210 g/L in the FEL.

In one embodiment, the VEL and LDS each comprise the same at least one sugar. In one embodiment, the VEL and LDS each comprise the same at least one sugar at the same predefined concentrations.

In one embodiment, the FEL, VEL and LDS each comprise the same at least one sugar. In one embodiment, the VEL and LDS each comprise the same at least one sugar at the same predefined concentrations and the FEL comprises the same at least one sugar at a different predefined concentration.

## 3. Set excipients: non-ionic surfactant

The non-ionic surfactant in the FEL, VEL or LDS can be selected from block copolymers, sorbitan esters, ethoxylated or propoxylated sorbitan esters, alkyl-polyglycosides (APG), alkoxylated mono- or di-alkylamines, fatty acid monoethanolamides (FAMA), fatty acid diethanolamides (FADA), ethoxylated fatty acid monoethanolamides (EFAM), propoxylated fatty acid monoethanolamides (PFAM), polyhydroxy alkyl fatty acid amides, or N-acyl N-alkyl derivatives of glucosamine (glucamides, GA, or fatty acid glucamide, FAGA), and combinations thereof.

The non-ionic surfactant in the FEL, VEL or LDS can be a high molecular weight non-ionic surfactant. The non-ionic surfactant can be a non-ionic triblock copolymer. The surfactant can be a non-ionic, hydrophilic, polyoxyethylene-polyoxypropylene block copolymer (or EO-PO block copolymer). The EO-PO block copolymers can include blocks of polyethylene oxide ($-CH_2CH_2O-$designated EO) and polypropylene oxide ($-CH_2CHCH_3O-$ designated PO). The PO block can be flanked by two EO blocks in a EOx-POy-Eox arrangement. Since the PO component is hydrophilic and the EO component is hydrophobic, the overall hydrophilicity, molecular weight and the surfactant properties of the copolymer can be adjusted by varying x and y in the EOx-POy-Eox block structure. In aqueous solutions, the EO-PO block copolymers will self-assemble into micelles with a PO core and a corona of hydrophilic EO groups.

The non-ionic surfactant in the FEL, VEL or LDS can be a poloxamer. Poloxamers are non-ionic triblock copolymers composed of a central hydrophobic chain of poly(propyleneoxide) flanked by two hydrophilic chains of poly(ethylene oxide). The length of the polymer blocks can be customized, leading to different poloxamers with slightly different properties. The non-ionic surfactant in the FEL, VEL or LDS can be Pluronic F127 (poloxamer 407), Pluronic F68 (poloxamer 403), Pluronic P123, Pluronic P85, other polyethylene oxide-polypropylene oxide (EO-PO) block copolymers of greater than 3,000-4,000 MW or combinations thereof.

The concentration of the non-ionic surfactant in the FEL, VEL or LDS can be the same. The concentration of the non-ionic surfactant in the FEL, VEL or LDS each can be with the range 2.0 to 12 g/L, 4 to 12 g/L, 6 to 12 g/L, 8 to 12 g/L, or 10 to 12 g/L. The concentration of a high molecular weight non-ionic surfactant in the FEL, VEL or LDS each can be with the range 2.0 to 12 g/L, 4 to 12 g/L, 6 to 12 g/L, 8 to 12 g/L, or 10 to 12 g/L. The concentration of a poloxamer in the FEL, VEL or LDS each can be with the range 2.0 to 12 g/L, 4 to 12 g/L, 6 to 12 g/L, 8 to 12 g/L, or 10 to 12 g/L. The concentration of Pluronic F127 (poloxamer 407) in the FEL, VEL or LDS each can be with the range 2.0 to 12 g/L, 4 to 12 g/L, 6 to 12 g/L, 8 to 12 g/L, or 10 to 12 g/L. The concentration of Pluronic F68 (poloxamer 403) in the FEL, VEL or LDS each can be with the range 2.0 to 12 g/L, 4 to 12 g/L, 6 to 12 g/L, 8 to 12 g/L, or 10 to 12 g/L. The concentration of Pluronic P123 in the FEL, VEL or LDS each can be with the range 2.0 to 12 g/L, 4 to 12 g/L, 6 to 12 g/L, 8 to 12 g/L, or 10 to 12 g/L. The concentration of a Pluronic P85 in the FEL, VEL or LDS each can be with the range 2.0 to 12 g/L, 4 to 12 g/L, 6 to 12 g/L, 8 to 12 g/L, or 10 to 12 g/L.

### 4. Set excipients: protein

The protein in the FEL, VEL or LDS can be any protein which is essentially inert and does not react with the virus. In particular, the protein does not affect the structure or infectivity of the virus. Thus, the protein can be a structural protein or a serum protein. The protein can be selected from an albumin, human serum albumin, collagen, hydrolyzed collagen, gelatin and hydrolyzed gelatin.

The concentration of the protein in the FEL, VEL or LDS can be the same. The concentration of protein in the FEL, VEL or LDS each can be within the range 0.5 to 1.5 g/L, 0.7 to 1.2 g/L, 0.8 to 1.0 g/L. The concentration of protein in the FEL, VEL or LDS each can be within the range 0.5 to 1.5 g/L, 0.7 to 1.2 g/L, 0.8 to 1.0 g/L. The concentration of albumin in the FEL, VEL or LDS each can be within the range 0.5 to 1.5 g/L, 0.7 to 1.2 g/L, 0.8 to 1.0 g/L. The concentration of human serum albumin in the FEL, VEL or LDS each can be within the range 0.5 to 1.5 g/L, 0.7 to 1.2 g/L, 0.8 to 1.0 g/L. The concentration of collagen in the FEL, VEL or LDS each can be within the range 0.5 to 1.5 g/L, 0.7 to 1.2 g/L, 0.8 to 1.0 g/L. The concentration of gelatin in the FEL and VEL each can be within the range 0.5 to 1.5 g/L, 0.7 to 1.2 g/L, 0.8 to 1.0 g/L.

### Combinations of excipients

In one embodiment, the VEL, LDS(s), and FEL each comprise a predefined concentration of at least four adjustable excipients, providing a LPC with a target concentration of the at least four adjustable excipients, wherein the at least four adjustable excipients are phosphate buffer, sodium chloride, potassium chloride, and trehalose.

In one embodiment, the VEL, LDS(s), and FEL each comprise a predefined concentration of four adjustable excipients, providing a LPC with a target concentration of the four adjustable excipients, wherein the four adjustable excipients in the VEL and LDS(s) are (i) a phosphate buffer comprising potassium dihydrogen phosphate within the range 0.9 to 1.2 mM and disodium hydrogen phosphate dihydrate within the range of 4 to 6 mM, (ii) sodium chloride in the range 100 to 130 mM, (iii) potassium chloride in the range 1 to 2 mM, and (iv) trehalose in the range of 120 to 160 g/L; and wherein the four adjustable excipients in the FEL are phosphate buffer comprising potassium dihydrogen phosphate within the range 3 to 4 mM and disodium hydrogen phosphate dihydrate within the range of 18 to 22 mM, sodium chloride in the range 20 to 50 mM, potassium chloride in the range of 4 to 6 mM, and trehalose in the range of 180 to 220 g/L.

In one embodiment, the VEL, LDS(s), and FEL each comprise a predefined concentration of four adjustable excipients and two set excipients, providing a LPC with a target concentration of the four adjustable excipients and two set excipients, wherein the four adjustable excipients in the VEL and LDS(s) are (i) a phosphate buffer comprising potassium dihydrogen phosphate within the range 0.9 to 1.2 mM and disodium hydrogen phosphate dihydrate within the range of 4 to 6 mM, (ii) sodium chloride in the range 100 to 130 mM, (iii) potassium chloride in the range 1 to 2 mM, and (iv) trehalose in the range of 120 to 160 g/L; and wherein the four adjustable excipients in the FEL are (i) a phosphate buffer comprising potassium dihydrogen phosphate within the range 3 to 4 mM and disodium hydrogen phosphate dihydrate within the range of 18 to 22 mM, (ii) sodium chloride in the range 20 to 50 mM, (iii) potassium chloride in the range of 4 to 6 mM, and (iv) trehalose in the range of 180 to 220 g/L; and wherein the two set excipients in the VEL, LDS(s) and FEL are F127 in the range of 2.0 to 12 g/L and HSA in the range of 0.5 to 1.5 g/L.

In one embodiment, the VEL, LDS(s), and FEL each comprise a predefined concentration of four adjustable excipients, providing a LPC with a target concentration of the four adjustable excipients, wherein the four adjustable excipients in the VEL and LDS(s) are (i) a phosphate buffer comprising potassium dihydrogen phosphate within the range 0.9 to 1.0 mM and disodium hydrogen phosphate dihydrate within the range of 5 to 6 mM, (ii) sodium chloride in the range 120 to 130 mM, (iii) potassium chloride in the range 1 to 1.5 mM, and (iv) trehalose in the range of 145 to 155 g/L; and wherein the four adjustable excipients in the FEL are (i) a phosphate buffer comprising potassium dihydrogen phosphate within the range 3.2 to 4 mM and disodium hydrogen phosphate dihydrate within the range of 20 to 22 mM, (ii) sodium chloride in the range 40 to 50 mM, (iii) potassium chloride in the range of 5 to 6 mM, and (iv) trehalose in the range of 200 to 210 g/L.

In one embodiment, the VEL, LDS(s), and FEL each comprise a predefined concentration of four adjustable excipients and two set excipients, providing a LPC with a target concentration of the four adjustable excipients and two set excipients, wherein the four adjustable excipients in the VEL and LDS(s) are (i) a phosphate buffer comprising potassium dihydrogen phosphate within the range 0.9 to 1.0 mM and disodium hydrogen phosphate dihydrate within the range of 5 to 6 mM, (ii) sodium chloride in the range 120 to 130 mM, (iii) potassium chloride in the range 1 to 1.5 mM, and (iv) trehalose in the range of 145 to 155 g/L; and wherein the four adjustable excipients in the FEL are (i) a phosphate buffer comprising potassium dihydrogen phosphate within the range 3.2 to 4 mM and disodium hydrogen phosphate dihydrate within the range of 20 to 22 mM, (ii) sodium chloride in the range 40 to 50 mM, (iii) potassium chloride in the range of 5 to 6 mM, and (iv) trehalose in the range of 200 to 210 g/L; and wherein the two set excipients in the VEL, LDS(s) and FEL are F127 in the range of 8 to 12 g/L and HSA in the range of 0.8 to 1.2 g/L.

## Volumes of VEL, LDS, FEL and LPC

It is in the nature of the invention that the volume of VEL, LDS and FEL are each smaller than the predefined target volume of the LPC. Furthermore, the volume of the VEL and LDS combined is smaller than the predefined target volume of the LPC. The combined volume of VEL, LDS and FEL is equal to the predefined target volume of the LPC. This applies to each batch of LPC. The VEL and FEL can be provided as stock solutions. The same volume of FEL can be used to prepare each batch of LPC, wherein each batch of LPC may comprise different batches of LDS(s). The volume of VEL will be adjusted depending on the volume of LDS(s) used.

Hence, in one embodiment, the VEL and FEL concentration of the at least one adjustable excipient do not change when different batches of LPC are prepared. This means that the same stock preparations of VEL and FEL can be used to prepare each batch of LPC.

The predefined target volume of the LPC can be any volume. For example, for each batch of LPC, the predefined target volume of the LPC may be between 10 L and 1000 L. In one embodiment, the predefined target volume of the LPC is between 10 L and 500 L, between 10 L and 250 L, between 10 L and 200 L, or between 10 L and 150 L,. In another embodiment, the predefined target volume of the LPC is at least 100 L. In one embodiment, the predefined target volume of the LPC is between 100 L and 500 L, between 100 L and 250 L, between 100 L and 200 L, or between 100 L and 150 L. In a preferred embodiment, the predefined target volume of the LPC is between 100 L and 250 L.

The LPC has a predefined target volume and is composed of a variable volume of at least one LDS ($^{v}V_{LDS(i)}$), the variable volume of VEL ($^{v}V_{VEL}$) and the fixed, predefined volume of FEL ($^{p}V_{FEL}$) according to the equation:

$$^{T}V_{LPC} = {}^{P}V_{FEL} + {}^{v}V_{VEL} + \sum_{i=1}^{\infty} V_{LDS(i)},$$

Hence, the volumes of FEL, VEL and each of the at least one LDS are smaller than the volume of LPC.

In one embodiment, the predefined volume of the FEL is less than the predefined target volume of the LPC.

In one embodiment, the predefined volume of the FEL is at least 10% of the predefined volume of the LPC. In one embodiment, the predefined volume of the FEL is between 10% and 90% of the predefined volume of the LPC. In one embodiment, the predefined volume of the FEL is between 10% and 80%, between 10% and 70%, between 10% and 60%, between 10% and 50%, or between 10% and 40% of the predefined volume of the LPC. In one embodiment, the predefined volume of the FEL is at least 20% of the predefined volume of the LPC. In one embodiment, the predefined volume of the FEL is between 20% and 90% of the predefined volume of the LPC. In one embodiment, the predefined volume of the FEL is between 20% and 80%, between 20% and 70%, between 20% and 60%, between 20% and 50%, or between 20% and 40% of the predefined volume of the LPC. In one embodiment, the predefined volume of the FEL is between 25% and 40% of the predefined volume of the LPC. In one embodiment, the predefined volume of the FEL is between 25% and 35% of the predefined volume of the LPC.

In one embodiment, the predefined volume of the FEL is at least 5% of the predefined volume of the LPC. In another embodiment, the predefined volume of the FEL is at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95% of the predefined volume of the LPC. In a preferred embodiment, the predefined volume of the FEL is at least 20% of the predefined volume of the LPC.

In most cases, several LDS(s) are used and the volume of each individual LDS is smaller than the volume of the VEL. Thus, in one embodiment, the variable volume of the LDS is less than the variable volume of VEL. In some cases, especially where an LDS has a low concentration of the biological active agent, the volume of an individual LDS may be larger than the volume of the VEL. Hence, in some embodiments, the variable volume of the LDS is larger than the variable volume of VEL.

The volume of the intermediate liquid (IL) is variable and can be expressed as $^{v}V_{IL} = {}^{p}V_{FEL} + {}^{v}V_{VEL}$. It is composed of one variable parameter, $^{v}V_{VEL}$, which is calculated according to $^{v}V_{VEL} = {}^{p}V_{(VEL+LDS)} - {}^{v}V_{LDS(total)}$ for each batch of LPC; and one fixed parameter, $^pV_{FEL}$. The sum of the volume of VEL and LDS, $^pV_{(VEL+LDS)}$ is also predefined and is chosen according to the process requirements.

In some cases where the concentration of the biological active agent in the at least one LDS is low, no VEL may be used. In this case, $^vV_{VEL} = 0$. Hence, in some embodiments, no VEL is used to make up the LPC. Hence, no VEL is added to the intermediate liquid (IL). In the case that $^vV_{VEL} = 0$, it follows that $^vV_{IL} = ^pV_{FEL}$.

In one embodiment, the sum of the variable volume of LDS and the variable volume of VEL is between 10% and 90% of the predefined volume of the LPC. In one embodiment, the sum of the variable volume of LDS and the variable volume of VEL is between 10% and 80%, between 10% and 70%, between 10% and 60%, between 10% and 50%, or between 10% and 40% of the predefined volume of the LPC. In one embodiment, the sum of the variable volume of LDS and the variable volume of VEL is between 20% and 90% of the predefined volume of the LPC. In one embodiment, the sum of the variable volume of LDS and the variable volume of VEL is between 20% and 80%, between 30% and 80%, between 40% and 80%, between 50% and 80%, or between 60% and 80% of the predefined volume of the LPC. In one embodiment, the sum of the variable volume of LDS and the variable volume of VEL is between 65% and 75% of the predefined volume of the LPC.

In one embodiment, the sum of the variable volume of LDS and the variable volume of VEL is at least 10% of the predefined volume of the LPC. In another embodiment, the sum of the variable volume of LDS and the variable volume of VEL is at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95% of the predefined volume of the LPC. In a preferred embodiment, the sum of the variable volume of LDS and the variable volume of VEL is at least 50% of the predefined volume of the LPC.

**Biological active agent**

The at least one LDS comprises one biological active agent at a variable concentration ($^V[A_i]_{LDS}$). According to the present invention, variable volumes of LDS are used to achieve the desired concentration of the biological active agent from each LDS in the LPC. A variable volume of the at least one LDS is mixed with an intermediate liquid (IL) comprising a predefined volume of FEL and a variable volume of VEL, to formulate a predefined volume of LPC. This applies to each batch of LPC.

In one embodiment, the LPC comprises at least one LDS. In one embodiment, the LPC comprises at least two, at least three, at least four, at least five, at least six, at least seven, at least eight, at least nine, or at least ten LDSs. In one embodiment, the LPC comprises between 1 and 20 LDSs, between 1 and 15 LDSs, between 1 and 10 LDSs, or between 1 and 5 LDSs. In one embodiment, the LPC comprises at least four LDSs. In one embodiment, the LPC comprises four LDSs.

In one embodiment, the biological active agent is a flavivirus. In one embodiment, the biological active agent is a dengue virus, preferably a live attenuated dengue virus.

In one embodiment, the LPC comprises at least one dengue virus serotype. In one embodiment, the LPC comprises at least two different dengue virus serotypes. In one embodiment, the LPC comprises at least three different dengue virus serotypes. In one embodiment, the LPC comprises four different dengue virus serotypes.

In one embodiment, the LPC comprises at least one chimeric dengue strain. In one embodiment, the LPC comprises at least two different chimeric dengue strains. In one embodiment, the LPC comprises at least three different chimeric dengue strains. In one embodiment, the LPC comprises at least four different chimeric dengue strains.

In one embodiment, the LPC comprises at least one dengue virus serotype strain and at least one chimeric dengue strain. In one embodiment, the LPC comprises at least one dengue virus serotype strain and at least two different chimeric dengue strains. In one embodiment, the LPC comprises at least one dengue virus serotype strain and at least three different chimeric dengue strains.

Preferably, the dengue virus strains and chimeras described herein are live attenuated dengue virus strains and chimeras.

In one embodiment, the LPC comprises four biological active agents, wherein the four biological active agents are:

(i) live attenuated dengue virus serotype 1,

(ii) live attenuated dengue virus serotype 2,

(iii) live attenuated dengue virus serotype 3, and

(iv) live attenuated dengue virus serotype 4.

In one embodiment, the LPC comprises four biological active agents, wherein the four biological active agents are:

(i) live attenuated chimeric dengue virus serotype 1,

(ii) live attenuated chimeric dengue virus serotype 2,

(iii) live attenuated chimeric dengue virus serotype 3, and

(iv) live attenuated chimeric dengue virus serotype 4.

In one embodiment, the LPC comprises four biological active agents, wherein the four biological active agents are:

(i) live attenuated chimeric dengue virus serotype 1,

(ii) live attenuated dengue virus serotype 2,

(iii) live attenuated chimeric dengue virus serotype 3, and

(iv) live attenuated chimeric dengue virus serotype 4.

In one embodiment, the LPC comprises four biological active agents, wherein the four biological active agents are:

(i) live attenuated dengue virus serotype 1,

(ii) live attenuated dengue virus serotype 2,

(iii) live attenuated dengue virus serotype 3, and

(iv) live attenuated dengue virus serotype 4;

wherein the dengue serotype 1 is a chimeric dengue serotype 2/1 strain, the dengue serotype 2 is a non-chimeric dengue serotype 2 strain, the dengue serotype 3 is a chimeric dengue serotype 2/3 strain and the dengue serotype 4 is a chimeric dengue serotype 2/4 strain;

wherein the dengue serotype 2 strain is derived from the wild type virus strain DEN-2 16681 and differs in at least three nucleotides from the wild type as follows:

a) 5'-noncoding region (NCR)-57 (nt-57)

b) NS1-53 Gly-to-Asp (nt-2579)

c) NS3-250 Glu-to-Val (nt-5270);

wherein the three chimeric dengue strains are

biological active agents, wherein the four biological active agents are derived from the serotype 2 strain by replacing the structural proteins prM and E from serotype 2 strain with the corresponding structural proteins from the other dengue serotypes, resulting in the following chimeric dengue strains:

- a DENV-2/1 chimera,
- a DENV-2/3 chimera, and
- a DENV-2/4 chimera.

Hence, in one embodiment, the LPC comprises the dengue serotype 2 strain (TDV-2), the DENV-2/1 chimera (TDV-1), the DENV-2/3 (TDV-3) chimera and the DENV-2/4 (TDV-4) chimera as described above.
In one embodiment, the chimeric dengue serotype 2/1 strain (TDV-1) comprises a nucleic acid sequence that is at least 70 % identical to the nucleic acid sequence according to SEQ ID NO: 1. In one embodiment, the chimeric dengue serotype 2/1 strain (TDV-1) comprises a nucleic acid sequence that is at least 75 %, at least 80%, at least 85%, at least 90%, at least 95%, at least 98%, or at least 99% identical to the nucleic acid sequence according to SEQ ID NO: 1.

In one embodiment, the chimeric dengue serotype 2/1 strain (TDV-1) comprises a nucleic acid sequence encoding for an amino acid sequence that is at least 70 % identical to the amino acid sequence according to SEQ ID NO: 2. In one embodiment, the chimeric dengue serotype 2/1 strain (TDV-1) comprises a nucleic acid sequence encoding for an amino acid sequence that is at least 75 %, at least 80%, at least 85%, at least 90%, at least 95%, at least 98%, or at least 99% identical to the amino acid sequence according to SEQ ID NO: 2.

In one embodiment, the a dengue serotype 2 strain (TDV-2) comprises a nucleic acid sequence that is at least 70 % identical to the nucleic acid sequence according to SEQ ID NO: 3. In one embodiment, the a dengue serotype 2 strain (TDV-2) comprises a nucleic acid sequence that is at least 75 %, at least 80%, at least 85%, at least 90%, at least 95%, at least 98%, or at least 99% identical to the nucleic acid sequence according to SEQ ID NO: 3.

In one embodiment, the dengue serotype 2 strain (TDV-2) comprises a nucleic acid sequence encoding for an amino acid sequence that is at least 70 % identical to the amino acid sequence according to SEQ ID NO: 4. In one embodiment, the dengue serotype 2 strain (TDV-2) comprises a nucleic acid sequence encoding for an amino acid sequence that is at least 75 %, at least 80%, at least 85%, at least 90%, at least 95%, at least 98%, or at least 99% identical to the amino acid sequence according to SEQ ID NO: 4.

In one embodiment, the chimeric dengue serotype 2/3 strain (TDV-3) comprises a nucleic acid sequence that is at least 70 % identical to the nucleic acid sequence according to SEQ ID NO: 5. In one embodiment, the chimeric dengue serotype 2/3 strain (TDV-3) comprises a nucleic acid sequence that is at least 75 %, at least 80%, at least 85%, at least 90%, at least 95%, at least 98%, or at least 99% identical to the nucleic acid sequence according to SEQ ID NO: 5.

In one embodiment, the chimeric dengue serotype 2/3 strain (TDV-3) comprises a nucleic acid sequence encoding for an amino acid sequence that is at least 70 % identical to the amino acid sequence according to SEQ ID NO: 6. In one embodiment, the chimeric dengue serotype 2/3 strain (TDV-3) comprises a nucleic acid sequence encoding for an amino acid sequence that is at least 75 %, at least 80%, at least 85%, at least 90%, at least 95%, at least 98%, or at least 99% identical to the amino acid sequence according to SEQ ID NO: 6.

In one embodiment, the chimeric dengue serotype 2/4 strain (TDV-4) comprises a nucleic acid sequence that is at least 70 % identical to the nucleic acid sequence according to SEQ ID NO: 7. In one embodiment, the chimeric dengue serotype 2/4 strain (TDV-4) comprises a nucleic acid sequence that is at least 75 %, at least 80%, at least 85%, at least 90%, at least 95%, at least 98%, or at least 99% identical to the nucleic acid sequence according to SEQ ID NO: 7.

In one embodiment, the chimeric dengue serotype 2/4 strain (TDV-4) comprises a nucleic acid sequence encoding for an amino acid sequence that is at least 70 % identical to the amino acid sequence according to SEQ ID NO: 8. In one embodiment, the chimeric dengue serotype 2/4 strain (TDV-4) comprises a nucleic acid sequence encoding for an amino acid sequence that is at least 75 %, at least 80%, at least 85%, at least 90%, at least 95%, at least 98%, or at least 99% identical to the amino acid sequence according to SEQ ID NO: 8.

In a preferred embodiment, TDV-1 is characterized by the nucleotide sequence of SEQ ID NO. 1, TDV-2 is characterized by the nucleotide sequence of SEQ ID NO. 3, TDV-3 is characterized by the nucleotide sequence of SEQ ID NO. 5, and/or TDV-4 is characterized by the nucleotide sequence of SEQ ID NO. 7. In a further preferred embodiment, TDV-1 is characterized by the amino acid sequence of SEQ ID NO. 2, TDV-2 is characterized by the amino acid sequence of SEQ ID NO. 4, TDV-3 is characterized by the amino acid sequence of SEQ ID NO. 6, and TDV-4 is characterized by the amino acid sequence of SEQ ID NO. 8. In a further preferred embodiment, TDV-1 is characterized by a nucleotide sequence encoding the amino acid sequence of SEQ ID NO. 2, TDV-2 is characterized by a nucleotide sequence encoding the amino acid sequence of SEQ ID NO. 4, TDV-3 is characterized by a nucleotide sequence encoding the amino acid sequence of SEQ ID NO. 6, and TDV-4 is characterized by a nucleotide sequence encoding the amino acid sequence of SEQ ID NO. 8.

**Table 4. Sequences of the TDV virus strains**

| SEQ ID NO. | dengue virus strain | sequence type |
|---|---|---|
| **SEQ ID NO. 1** | TDV-1 (DENV-2/1 chimera) | nucleotide sequence |
| **SEQ ID NO. 2** | TDV-1 (DENV-2/1 chimera) | amino acid sequence |
| **SEQ ID NO. 3** | TDV-2 (DENV-2) | nucleotide sequence |
| **SEQ ID NO. 4** | TDV-2 (DENV-2) | amino acid sequence |
| **SEQ ID NO. 5** | TDV-3 (DENV-2/3 chimera) | nucleotide sequence |
| **SEQ ID NO. 6** | TDV-3 (DENV-2/3 chimera) | amino acid sequence |
| **SEQ ID NO. 7** | TDV-4 (DENV-2/4 chimera) | nucleotide sequence |

(continued)

| SEQ ID NO. | dengue virus strain | sequence type |
|---|---|---|
| SEQ ID NO. 8 | TDV-4 (DENV-2/4 chimera) | amino acid sequence |

The volume of each of the at least one LDS to be added is calculated according to the predefined target volume of the LPC and the predefined target concentration of the biological active agent in the LPC.

In one embodiment, the biological active agent is a flavivirus. In one embodiment, the biological active agent is a dengue virus. The dengue virus may be at least one dengue virus serotype strain. The dengue virus may be at least one chimeric dengue strain.

In one embodiment, the LPC comprises at least one dengue virus serotype strain. In one embodiment, the LPC comprises at least two different dengue virus serotype strains. In one embodiment, the LPC comprises at least three different dengue virus serotype strains. In one embodiment, the LPC comprises four different dengue virus serotype strains, such as .g. four different live attenuated dengue virus serotype strains.

In one embodiment, the LPC comprises at least one chimeric dengue strain. In one embodiment, the LPC comprises at least two different chimeric dengue strains. In one embodiment, the LPC comprises at least three different chimeric dengue strains. In one embodiment, the LPC comprises at least four different live attenuated dengue strains, such as e.g. four different live attenuated strains, for example four different live attenuated dengue strains with each strain being e.g. either a chimeric and/or non-chimeric dengue strain, in particular such as TAK-003.

Typically, the target concentration of the at least one biological active agent in the LPC is predefined according to the specific requirements of the LPC product.

In one embodiment, the biological active agent is a flavivirus and the target concentration of the flavivirus in the LPC is a titer of at least $10^3$ PFU/ml. In another embodiment, the biological active agent is a flavivirus and the target concentration of the flavivirus in the LPC is a titer of at least $10^4$ PFU/ml. In another embodiment, the biological active agent is a flavivirus and the target concentration of the flavivirus in the LPC is a titer of at least $10^5$ PFU/ml.

In one embodiment, the biological active agent is a live, attenuated dengue virus strain, preferably a dengue virus serotype strain or chimeric dengue virus strain, and the target concentration of the live, attenuated dengue virus strain in the LPC is a titer of at least $3 \log_{10}$ PFU/ml. In another embodiment, the biological active agent is a live, attenuated dengue virus strain, preferably a dengue virus serotype strain or chimeric dengue virus strain, and the target concentration of the live, attenuated dengue virus strain in the LPC is a titer of at least $4 \log_{10}$ PFU/ml. In another embodiment, the biological active agent is a live, attenuated dengue virus strain, preferably a dengue virus serotype strain or chimeric dengue virus strain, and the target concentration of the live, attenuated dengue virus strain in the LPC is a titer of at least $5 \log_{10}$ PFU/ml.

In one embodiment, the batch mixing process is a large-scale batch process. "Large-scale" also called "production scale" or "manufacturing scale" as used herein refers to provision of the biological active agent, e.g. a flavivirus at a large scale as opposed to a laboratory scale or small scale.

Hence, in accordance with the large-scale manufacturing process, the target concentration of the biological active agent, e.g. a flavivirus or dengue virus, is prepared in a large volume. Thus, the total amount of the biological active agent is high compared to small-scale, e.g. laboratory scale, processes. For example, the target titer for one batch of LPC may be about $6 \log_{10}$ PFU/ml in 100ml, which means about $11 \log_{10}$ PFU total of virus.

The LPC is made up of at least one batch of LDS which, in accordance with the large-scale manufacturing process, has a high concentration of the biological active agent, e.g. a flavivirus or dengue virus, manufactured in a large volume. For example, if four LDSs are combined to make up one batch of LPC, they may each have a different volume, such that $^vV_{LDS1} + {}^vV_{LDS2} + {}^vV_{LDS3} + {}^vV_{LDS4} + {}^pV_{FEL} = {}^pV_{LPC}$.

In one embodiment, the large-scale titer of the live, attenuated dengue virus strain in the LDS can be at least $6 \log_{10}$ PFU/ml. In most cases, the large-scale titer of the live, attenuated dengue virus strain in the LDS can be between $6.0 \log_{10}$ PFU/ml and $9.0 \log_{10}$ PFU/ml. Exemplarily, each batch of LDS used to make up one batch of LPC of a large-scale process can have a volume of up to 30 L and a titer of between $6.0 \log_{10}$ PFU/ml and $9.0 \log_{10}$ PFU/ml. Hence, in one embodiment, the total virus content of each batch of LDS used to make up one batch of LPC can be between $10.4 \log_{10}$ PFU and $13.5 \log_{10}$ PFU.

In another example, each batch of LDS used to make up one batch of LPC of a large-scale process can have a volume of at least 50 ml and a titer of between $6.0 \log_{10}$ PFU/ml and $9.0 \log_{10}$ PFU/ml. Hence, in one embodiment, the total virus content of each batch of LDS used to make up one batch of LPC can be between $7.5 \log_{10}$ PFU and $10.8 \log_{10}$ PFU.

Hence, in one embodiment, the total virus content of each batch of LDS used to make up one batch of LPC can be between $7.5 \log_{10}$ PFU and $13.5 \log_{10}$ PFU.

In one embodiment, the large-scale target titer of the live, attenuated dengue virus strain in the LPC can be at least $4 \log_{10}$ PFU/ml. In most cases, the large-scale target titer of the live, attenuated dengue virus strain in the LPC can be between $5.0 \log_{10}$ PFU/ml and $7.0 \log_{10}$ PFU/ml. Exemplarily, each batch of LPC of a large-scale process can have a volume of between 100 and 150L and a target titer of between $5.0 \log_{10}$ PFU/ml and $7.0 \log_{10}$ PFU/ml. Hence, in one embodiment, the total virus content of each batch of LPC can be between $9 \log_{10}$ PFU and $12.18 \log_{10}$ PFU. In another embodiment, the total virus content of each batch of LPC can be between $10 \log_{10}$ PFU and $12.18 \log_{10}$ PFU. In another embodiment, the total virus content of each batch of LPC can be between $10.3 \log_{10}$ PFU and $12.18 \log_{10}$ PFU. In another embodiment, the total virus content of each batch of LPC can be between $10.3 \log_{10}$ PFU and $12.07 \log_{10}$ PFU.

In one embodiment, the large-scale batch process comprises processing multiple batches with varying LDS concentrations. For example, at least two batches of $LDS_{1.j}$ ($LDS_{1.1}$, $LDS_{1.2}$ and so on) each comprising a biological active agent A1 at a variable concentration ${}^V[A_1]_{LDS1.j}$.

In one embodiment, the large-scale batch process comprises processing at least two batches of $LDS_{i.j}$ each comprising a biological active agent Ai at a variable concentration ${}^V[A_i]_{LDSi.j}$. In another embodiment, the large-scale batch process comprises processing at least three batches, at least four batches, at least five batches, at least six batches, at least seven batches, at least eight batches, at least nine batches, at least ten batches of $LDS_{i.j}$ each comprising a biological active agent Ai at a variable concentration ${}^V[A_i]_{LDSi.j}$;

Hence, in one embodiment, the large-scale batch process comprises processing at least two batches of $LDS_{1.j}$ each comprising a biological active agent A1 at a variable concentration ${}^V[A_1]_{LDS1.j}$; at least two batches of $LDS_{2.j}$ each comprising a biological active agent A2 at a variable concentration ${}^V[A_2]_{LDS2.j}$; at least two batches of $LDS_{3.j}$ each comprising a biological active agent A3 at a variable concentration ${}^V[A_3]_{LDS3.j}$ and at least two batches of $LDS_{4.j}$ each comprising a biological active agent A4 at a variable concentration ${}^V[A_4]_{LDS4.j}$.

In one embodiment, the large-scale batch process comprises processing at least two batches of $LDS_{1.j}$ each comprising a dengue serotype 1 (D1) at a variable concentration ${}^V[D_1]_{LDS1.j}$; at least two batches of $LDS_{2.j}$ each comprising a dengue serotype 2 (D2) at a variable concentration ${}^V[D_2]_{LDS2.j}$; at least two batches of $LDS_{3.j}$ each comprising a dengue serotype 3 (D3) at a variable concentration ${}^V[D_3]_{LDS3.j}$ and at least two batches of $LDS_{4.j}$ each comprising dengue serotype 4 (D4) at a variable concentration ${}^V[D_4]_{LDS4.j}$.

In one embodiment, the large-scale batch process comprises processing at least three batches, at least four batches, at least five batches, at least six batches, at least seven batches, at least eight batches, at least nine batches, at least ten batches of $LDS_{1.j}$ each comprising a dengue serotype 1 (D1) at a variable concentration ${}^V[D_1]_{LDS1.j}$; at least three batches, at least four batches, at least five batches, at least six batches, at least seven batches, at least eight batches, at least nine batches, at least ten batches of $LDS_{2.j}$ each comprising a dengue serotype 2 (D2) at a variable concentration ${}^V[D_2]_{LDS2.j}$; at least three batches, at least four batches, at least five batches, at least six batches, at least seven batches, at least eight batches, at least nine batches, at least ten batches of $LDS_{3.j}$ each comprising a dengue serotype 3 (D3) at a variable concentration ${}^V[D_3]_{LDS3.j}$ and at least three batches, at least four batches, at least five batches, at least six batches, at least seven batches, at least eight batches, at least nine batches, at least ten batches of $LDS_{4.j}$ each comprising dengue serotype 4 (D4) at a variable concentration ${}^V[D_4]_{LDS4.j}$.

In one embodiment, the large-scale batch process comprises processing at least three batches, at least four batches, at least five batches, at least six batches, at least seven batches, at least eight batches, at least nine batches, at least ten batches each of $LDS_{1.j}$, each comprising a dengue serotype 1 (D1) at a variable concentration ${}^V[D_1]_{LDS1.j}$; of $LDS_{2.j}$ each comprising a dengue serotype 2 (D2) at a variable concentration ${}^V[D_2]_{LDS2.j}$; of $LDS_{3.j}$ each comprising a dengue serotype 3 (D3) at a variable concentration ${}^V[D_3]_{LDS3.j}$ and of $LDS_{4.j}$ each comprising dengue serotype 4 (D4) at a variable concentration ${}^V[D_4]_{LDS4.j}$.

In one embodiment, the process of the invention comprises in step 1 providing one LDS comprising one live, attenuated dengue virus strain at a variable concentration of ${}^V[A_1]_{LDS}$, and in step 2 mixing the one LDS at a variable volume of ${}^{vv}V_{LDS(1)}$ to provide a LPC with a target concentration of the live, attenuated dengue virus strain of ${}^T[A_1]_{LPC}$, preferably wherein the live, attenuated dengue virus strain is a chimeric dengue serotype 2/1 strain (TDV-1).

In one embodiment, the process of the invention comprises in step 1 providing one LDS comprising one live, attenuated dengue virus strain at a variable concentration of ${}^V[A_1]_{LDS}$, and in step 2 mixing the one LDS at a variable volume of ${}^{vv}V_{LDS(1)}$ to provide a LPC with a target concentration of the live, attenuated dengue virus strain of ${}^T[A_1]_{LPC}$, preferably wherein the live, attenuated dengue virus strain is a dengue serotype 2 strain (TDV-2).

In one embodiment, the process of the invention comprises in step 1 providing one LDS comprising one live, attenuated dengue virus strain at a variable concentration of ${}^V[A_1]_{LDS}$, and in step 2 mixing the one LDS at a variable volume of ${}^{vv}V_{LDS(1)}$ to provide a LPC with a target concentration of the live, attenuated dengue virus strain of ${}^T[A_1]_{LPC}$, preferably wherein the live, attenuated dengue virus strain is a chimeric dengue serotype 2/3 strain (TDV-3).

In one embodiment, the process of the invention comprises in step 1 providing one LDS comprising one live, attenuated dengue virus strain at a variable concentration of ${}^V[A_1]_{LDS}$, and in step 2 mixing the one LDS at a variable volume of ${}^{vv}V_{LDS(1)}$ to provide a LPC with a target concentration of the live, attenuated dengue virus strain of ${}^T[A_1]_{LPC}$, preferably wherein the live, attenuated dengue virus strain is a chimeric dengue serotype 2/4 strain (TDV-4).

In one embodiment, the process of the invention comprises in step 1 providing two LDSs each comprising one live, attenuated dengue virus strain at a variable concentration of ${}^V[A_1]_{LDS}$ and ${}^V[A_2]_{LDS}$, and in step 2 mixing the two LDSs each at a variable volume of ${}^{vv}V_{LDS(1)}$ and ${}^{vv}V_{LDS(2)}$ to provide a LPC with a target concentration of the two live, attenuated dengue virus strains of $^T[A_1]_{LPC}$ and $^T[A_2]_{LPC}$, preferably wherein the two live, attenuated dengue virus strains are selected from a chimeric dengue serotype 2/1 strain (TDV-1), a dengue serotype 2 strain (TDV-2), a chimeric dengue serotype 2/3 strain (TDV-3), and a chimeric dengue serotype 2/4 strain (TDV-4), which can be combined in any pair-wise combination. For example, the two LDSs can comprise TDV-1 and TDV-2, or TDV-1 and TDV-3, and so on.

In one embodiment, the process of the invention comprises in step 1 providing three LDSs each comprising one live, attenuated dengue virus strain at a variable concentration of $^V[A_1]_{LDS}$, $^V[A_2]_{LDS}$ and $^V[A_3]_{LDS}$ and in step 2 mixing the three LDSs each at a variable volume of $^{vV}_{LDS(1)}$, $^{vV}_{LDS(2)}$ and $^{vV}_{LDS(3)}$ to provide a LPC with a target concentration of the two live, attenuated dengue virus strains of $^T[A_1]_{LPC}$, $^T[A_2]_{LPC}$. and $^T[A_3]_{LPC}$, preferably wherein the three live, attenuated dengue virus strains are selected from a chimeric dengue serotype 2/1 strain (TDV-1), a dengue serotype 2 strain (TDV-2), a chimeric dengue serotype 2/3 strain (TDV-3), and a chimeric dengue serotype 2/4 strain (TDV-4), which can be combined in any combination. For example, the three LDSs can comprise TDV-1, TDV-2 and TDV-3, or TDV-1, TDV-3 and TDV-4, and so on.

In one embodiment, the process of the invention comprises in step 1 providing four LDSs each comprising one live, attenuated dengue virus strain at a variable concentration of $^V[A_1]_{LDS}$, $^V[A_2]_{LDS}$, $^V[A_3]_{LDS}$ and $^V[A_4]_{LDS}$ and in step 2 mixing the four LDSs each at a variable volume of $^{vV}_{LDS(1)}$, $^{vV}_{LDS(2)}$, $^{vV}_{LDS(3)}$ and $^{vV}_{LDS(4)}$ to provide a LPC with a target concentration of the four live, attenuated dengue virus strains of $^T[A_1]_{LPC}$, $^T[A_2]_{LPC}$, $^T[A_3]_{LPC}$ and $^T[A_4]_{LPC}$, preferably wherein the four live, attenuated dengue virus strains are a chimeric dengue serotype 2/1 strain (TDV-1), a dengue serotype 2 strain (TDV-2), a chimeric dengue serotype 2/3 strain (TDV-3), and a chimeric dengue serotype 2/4 strain (TDV-4).

In one embodiment, the VEL, four LDSs, and FEL each comprise a predefined concentration of four adjustable excipients and each LDS comprises one biological active agent, providing a LPC with a target concentration of the four adjustable excipients and four biological active agents; wherein the four adjustable excipients in the VEL and LDS(s) are phosphate buffer comprising potassium dihydrogen phosphate within the range 0.9 to 1.2 mM and disodium hydrogen phosphate dihydrate within the range of 4 to 6 mM, sodium chloride in the range 100 to 130 mM , potassium chloride in the range 1 to 2 mM, and trehalose in the range of 120 to 160 g/L; and wherein the four adjustable excipients in the FEL are phosphate buffer comprising potassium dihydrogen phosphate within the range 3 to 4 mM and disodium hydrogen phosphate dihydrate within the range of 18 to 22 mM , sodium chloride in the range 20 to 50 mM , potassium chloride in the range of 4 to 6 mM, and trehalose in the range of 180 to 220 g/L; and wherein the four biological active agents are a chimeric dengue serotype 2/1 strain (TDV-1), a dengue serotype 2 strain (TDV-2), a chimeric dengue serotype 2/3 strain (TDV-3), and a chimeric dengue serotype 2/4 strain (TDV-4).

In one embodiment, the VEL, four LDSs, and FEL each comprise a predefined concentration of four adjustable excipients and two set excipients and each LDS comprises one biological active agent, providing a LPC with a target concentration of the four adjustable excipients, two set excipients and four biological active agents; wherein the four adjustable excipients in the VEL and LDS(s) are phosphate buffer comprising potassium dihydrogen phosphate within the range 0.9 to 1.2 mM and disodium hydrogen phosphate dihydrate within the range of 4 to 6 mM, sodium chloride in the range 100 to 130 mM, potassium chloride in the range 1 to 2 mM, and trehalose in the range of 120 to 160 g/L; and wherein the four adjustable excipients in the FEL are phosphate buffer comprising potassium dihydrogen phosphate within the range 3 to 4 mM and disodium hydrogen phosphate dihydrate within the range of 18 to 22 mM, sodium chloride in the range 20 to 50 mM , potassium chloride in the range of 4 to 6 mM, and trehalose in the range of 180 to 220 g/L; and wherein the two set excipients in the VEL, LDS(s) and FEL are F127 in the range of 2.0 to 12 g/L and HSA in the range of 0.5 to 1.5 g/L and wherein the four biological active agents are a chimeric dengue serotype 2/1 strain (TDV-1), a dengue serotype 2 strain (TDV-2), a chimeric dengue serotype 2/3 strain (TDV-3), and a chimeric dengue serotype 2/4 strain (TDV-4).

## EXAMPLES

The following example is included to demonstrate certain aspects and embodiments of the invention as described in the claims. It should be appreciated by those of skill in the art, however, that the following description is illustrative only and should not be taken in any way as a restriction of the invention.

## EXAMPLE 1 (MIXING PROCESS FOR A DENGUE VIRUS VACCINE)

In the following, as an example, a liquid pharmaceutical composition (LPC) with a batch size of 127 L with predefined titers and predefined concentrations of excipients is prepared from 4 dengue liquid drug substances (LDSs) by admixing a variable excipient liquid (VEL) and a fixed excipient liquid (FEL). The predefined titer of the LPC is the formulation target titer.

Table 1: Determination of the volume of each LDS required to obtain a target titer of biological active agent in 127L LPC from each individual liquid drug substance (serotypes dengue-1, dengue-2, dengue-3, dengue-4)

| Serotype /LDS | Target Titer (Log$_{10}$PFU/mL) in LPC (127L) | LDS Titer (Log$_{10}$PFU/mL) | Volume of each serotype added (mL) |
|---|---|---|---|
| dengue-1 (D$_1$) | 5 | 8[1] | 127 |
| dengue-2 (D$_2$) | 6 | 7.5[1] | 4016.1 |
| dengue-3 (D$_3$) | 6 | 8.5[1] | 401.6 |
| dengue-4 (D$_4$) | 5.5 | 7.5[1] | 1270 |
| Total | | | 5814.7 |

[1] Example concentrations of each of the biological active agents present in each of the four different liquid drug substances (dengue-1, dengue-2, dengue-3 and dengue-4) individually.

Table 2: Determination of volumes to be mixed to form the LPC

| Liquid | VEL | LDS | FEL | LPC |
|---|---|---|---|---|
| Potassium dihydrogen phosphate (mM) | 0.939 | 0.939 | 3.76 | 1.76 |
| Disodium hydrogen phosphate, dihydrate (mM) | 5.33 | 5.33 | 21.4 | 10.0 |
| *Total concentration of phosphate ions (mM)[2]* | *6.27* | *6.27* | *25.2* | *11.76* |
| Sodium chloride (mM) | 124 | 124 | 41.6 | 100.0 |
| Potassium chloride (mM) | 1.44 | 1.44 | 5.76 | 2.7 |
| Trehalose dihydrate (g/L) | 150 | 150 | 204 | 165.8 |
| F127 (g/L) | 10.0 | 10.0 | 10.0 | 10.0 |
| HSA (g/L) | 1.00 | 1.00 | 1.00 | 1.00 |
| Volumes of VEL and LDS | 84.1853 L | 5.8147 L | | |
| Combined volume VEL and LDS, and FEL | 90 L | | 37 L | |
| Combined volume of VEL, LDS and FEL = total volume of LPC | 127 L | | | 127 L |

The pH of the LPC is between 7.0 and 8.5
[2]Refers to ions in both their protonated and basic forms.

For the example LPC with a batch size of 127 L, a maximum LDSs + VEL volume of 90 L was chosen in order to account for a range of possible concentrations of the biological active agent, e.g. virus titers. Accordingly, a FEL volume of 37 L was chosen.

The titer value for each LDS is naturally variable due to the underlying biological process. Due to the variability in titer values of different batches of LDS, in order to achieve the desired end titer of each dengue serotype (TDV-1, TDV-2, TDV-3 and TDV-4) in the liquid pharmaceutical composition (LPC), different volumes of each of the LDSs for each dengue serotype must be added. These volumes change from batch to batch due to the changing titers resulting from the upstream process. Since the excipient concentrations in the VEL are, according to the present invention, defined to be the same as in the LDSs, various total volumes of LDSs can be added to the VEL in order to obtain a predefined volume (e.g. 90 L) of the LDSs and the VEL, while the excipient concentrations are constant. The predefined volume of the LDSs and the VEL (e.g. 90 L) can afterwards be combined with a predefined volume of FEL (e.g. 37 L) with a defined excipient concentration predefined to obtain the target concentration of excipients and the target titers in the LPC.

Thus, from batch to batch only the volume of VEL has to be adjusted to the required volume of LDSs in a specific batch depending on the titers of each of the LDSs, whereas all other volumes and concentrations have to be determined only once and do not change from batch to batch. In particular, the VEL and FEL composition and concentration do not change and can be prepared as stock solutions. The adjusting of the volume of VEL is a simple adjustment.

The process follows the following steps:

(1) First, the required volume of each LDS is determined for each virus serotype:

$$Required\ volume = \frac{10^{Target\ Titer} \times 127\ L}{10^{LDS\ titer}}$$

(2) The required volume of VEL is then determined:

90 *L - Total volume of LDSs (all serotypes) = Volume of VEL required*

(3) Next, the determined volume of VEL is mixed with 37 L of FEL to form an Intermediate Liquid (IL).

(4) Finally the appropriate quantity of IL is added along with the required volume of each of the LDSs (which may have been combined) to

form the final pharmaceutical composition (LPC)

See figure 1 for a schematic diagram showing the above process.

The application further comprises the following items:

Item 1: A batch mixing process for preparing a liquid pharmaceutical composition (LPC) comprising at least one biological active agent and at least one adjustable excipient, wherein the at least one biological active agent has a target concentration in the LPC ($^T[A_i]_{LPC}$) and the at least one adjustable excipient has a target concentration in the LPC ($^T[E_x]_{LPC}$), the process comprising at least two steps:

step 1: providing

- a variable excipient liquid (VEL) comprising the at least one adjustable excipient at a predefined concentration ($^P[E_x]_{VEL}$),
- one or more liquid drug substances (LDS) comprising one biological active agent at a variable concentration ($^V[A_i]_{LDS}$) and the at least one adjustable excipient at a predefined concentration ($^P[E_x]_{LDS}$), and
- a fixed excipient liquid (FEL) comprising the at least one adjustable excipient at a predefined concentration ($^P[E_x]_{FEL}$), wherein the predefined concentration of each of the adjustable excipients is defined in accordance with equation 1:

$$^P[E_x]_{VEL} = {}^P[E_x]_{LDS} \neq {}^P[E_x]_{FEL} \tag{1}$$

wherein $^P[E_x]_{LDS}$ is predefined by the process of manufacture of LDS and $^P[E_x]_{FEL}$ is further defined by the conditions of step 2

step 2: combining

- all of the LDSs each at a variable volume ($^{VV}L_{DS(i)}$),
- the VEL at a variable volume ($^{VV}_{VEL}$), and
- the FEL at a predefined volume ($^PV_{FEL}$),

to form the LPC with a target volume ($^TV_{LPC}$) and with a target concentration of all of the biological active agents and all of the adjustable excipients, wherein the variable volume of each LDS is defined according to equation 2:

$$^vV_{LDS(i)} = \frac{^T[A_i]_{LPC}}{^V[A_i]_{LDS}} \times {}^TV_{LPC}, \tag{2}$$

the combined total volume ($^PV_{(VEL+LDS)}$) of all LDSs ( $^{VV}L_{DS(total)} = \sum_{i=1}^{\infty} V_{LDS(i)}$ ) and the VEL is predefined, and the variable volume of the VEL is defined according to equation 3:

$$^vV_{VEL} = {}^pV_{(VEL+LDS)} - {}^vV_{LDS(total)}, \tag{3}$$

and the predefined volume of the FEL ($^PV_{FEL}$) is defined according to equation 4:

$$^pV_{FEL} = {}^TV_{LPC} - {}^pV_{(VEL+LDS)}, \tag{4}$$

and the pre-defined concentration of each of the adjustable excipients in the FEL is defined according to equation 5:

$$^{p}[E_x]_{FEL} = \frac{^{T}[E_x]_{LPC} \times {}^{T}V_{LPC} - {}^{p}V_{(VEL+LDS)} \times {}^{p}[E_x]_{VEL}}{^{p}V_{FEL}} \qquad (5)$$

Item 2: The batch mixing process of item 1, comprising in step 1 providing one LDS comprising one biological active agent at a variable concentration of $^{V}[A_1]_{LDS}$, and in step 2 mixing the one LDS at a variable volume of $^{vV}_{LDS(1)}$ to provide a LPC with a target concentration of one biological active agent of $^{T}[A_1]_{LPC}$.

Item 3: The batch mixing process of item 1, comprising in step 1 providing two LDSs each comprising one biological active agent at a variable concentration of $^{V}[A_1]_{LDS}$ and $^{V}[A_2]_{LDS}$, and in step 2 mixing the two LDSs each at a variable volume of $^{vV}_{LDS(1)}$ and $^{vV}_{LDS(2)}$ to provide a LPC with a target concentration of the two biological active agents of $^{T}[A_1]_{LPC}$ and $^{T}[A_2]_{LPC}$.

Item 4: The batch mixing process of item 1, comprising in step 1 providing three LDSs each comprising one biological active agent at a variable concentration of $^{V}[A_1]_{LDS}$, $^{V}[A_2]_{LDS}$ and $^{V}[A_3]_{LDS}$ and in step 2 mixing the three LDSs each at a variable volume of $^{vV}_{LDS(1)}$, $^{vV}_{LDS(2)}$ and $^{vV}_{LDS(3)}$ to provide a LPC with a target concentration of the three biological active agents of $^{T}[A_1]_{LPC}$, $^{T}[A_2]_{LPC}$ and $^{T}[A_3]_{LPC}$.

Item 5: The batch mixing process of item 1, comprising in step 1 providing four LDSs each comprising one biological active agent at a variable concentration of $^{V}[A_1]_{LDS}$, $^{V}[A_2]_{LDS}$, $^{V}[A_3]_{LDS}$ and $^{V}[A_4]_{LDS}$ and in step 2 mixing the four LDSs each at a variable volume of $^{vV}_{LDS(1)}$, $^{vV}_{LDS(2)}$, $^{vV}_{LDS(3)}$ and $^{vV}_{LDS(4)}$ to provide a LPC with a target concentration of the four biological active agents of $^{T}[A_1]_{LPC}$, $^{T}[A_2]_{LPC}$, $^{T}[A_3]_{LPC}$ and $^{T}[A_4]_{LPC}$.

Item 6: The batch mixing process according to any one of the preceding items, wherein in step 2 the component liquids are combined in the following order:

    a. the FEL and the VEL are first combined to provide an intermediate liquid (IL),
    b. the IL and the at least one LDS are then subsequently combined to provide the LPC.

Item 7: The batch mixing process of any one of the preceding items for preparing a LPC, wherein

the VEL, LDS(s), and FEL each comprise a predefined concentration of one adjustable excipient

-   $^{P}[E_a]_{VEL}$ in the VEL,
-   $^{P}[E_a]_{LDS}$ in the LDS and
-   $P[E_a]_{FEL}$ in the FEL,

providing a LPC with a target concentration of the one adjustable excipient $^{T}[E_a]_{LPC}$; or wherein
the VEL, LDS(s), and FEL each comprise a predefined concentration of two adjustable excipients

-   $^{P}[E_a]_{VEL}$ and $^{P}[E_b]_{VEL}$ in the VEL,
-   $^{P}[E_a]_{LDS}$ and $^{P}[E_b]_{LDS}$ in the LDS and
-   $^{P}[E_a]_{FEL}$ and $^{P}[E_b]_{FEL}$ in the FEL,

providing a LPC with a target concentration of the two adjustable excipients $^{T}[E_a]_{LPC}$ and $^{T}[E_b]_{LPC}$; or wherein
the VEL, LDS(s), and FEL each comprise a predefined concentration of three adjustable excipients

-   $^{P}[E_a]_{VEL}$, $^{P}[E_b]_{VEL}$ and $^{P}[E_c]_{VEL}$ in the VEL,
-   $^{P}[E_a]_{LDS}$, $^{P}[E_b]_{LDS}$ and $^{P}[E_c]_{LDS}$ in the LDS and
-   $^{P}[E_a]_{FEL}$, $^{P}[E_b]_{FEL}$ and $^{P}[E_c]_{FEL}$ in the FEL;

providing a LPC with a target concentration of the three adjustable excipients $^{T}[E_a]_{LPC}$, $T[E_b]_{LPC}$ and $^{T}[E_c]_{LPC}$; or wherein
the VEL, LDS(s), and FEL each comprise a predefined concentration of four adjustable excipients

- $^P[E_a]_{VEL}$, $^P[E_b]_{VEL}$, $^P[E_c]_{VEL}$ and $^P[E_d]_{VEL}$ in the VEL,
- $^P[E_a]_{LDS}$, $^P[E_b]_{LDS}$, $^P[E_c]_{LDS}$ and $^P[E_d]_{LDS}$ in the LDS and
- $^P[E_a]_{FEL}$, $^P[E_b]_{FEL}$, $^P[E_c]_{FEL}$ and $^P[E_d]_{FEL}$ in the FEL;

providing a LPC with a target concentration of the four adjustable excipients $^T[E_a]_{LPC}$, $^T[E_b]_{LPC}$, $^T[E_c]_{LPC}$ and $^T[E_d]_{LPC}$.

Item 8: The batch mixing process of any one of the preceding items, wherein the at least one adjustable excipient is selected from the group of carbohydrates, salts and buffer salts.

Item 9: The batch mixing process of any one of the preceding items, wherein the VEL, LDS(s), and FEL further comprise at least one set excipient.

Item 10: The batch mixing process of item 9, wherein the at least one set excipient is selected from the group of non-ionic surfactants and albumins.

Item 11: The batch mixing process of item 9 or 10, wherein the VEL, LDS(s), FEL each comprise a predefined concentration of four adjustable excipients

- $^P[E_a]_{VEL}$, $^P[E_b]_{VEL}$, $^P[E_c]_{VEL}$ and $^P[E_d]_{VEL}$ in the VEL,
- $^P[E_a]_{LDS}$, $^P[E_b]_{LDS}$, $^P[E_c]_{LDS}$ and $^P[E_d]_{LDS}$ in the LDS and
- $^P[E_a]_{FEL}$, $^P[E_b]_{FEL}$, $^P[E_c]_{FEL}$ and $^P[E_d]_{FEL}$ in the FEL; and

a predefined concentration of two set excipients,
providing a LPC with a target concentration of four adjustable excipients $^T[E_a]_{LPC}$, $^T[E_b]_{LPC}$, $^T[E_c]_{LPC}$ and $^T[E_d]_{LPC}$; and
two set excipients.

Item 12: The batch mixing process of item 11, wherein the adjustable excipients are phosphate buffer, sodium chloride, potassium chloride, and trehalose, and the set excipients are F127 and HSA.

Item 13: The batch mixing process of any one of the previous items, wherein the one biological active agent is a virus, such as a live attenuated virus.

Item 14: The batch mixing process of item 13, wherein the virus is a flavivirus, such as a dengue virus.

Item 15: The batch mixing process of item 14, comprising in step 1 providing four LDSs each comprising one of the four serotypes of dengue virus $D_1$ $D_2$ $D_3$ and $D_4$ at a variable concentration of $^V[D_1]_{LDS}$, $^V[D_2]_{LDS}$, $^V[D_3]_{LDS}$, and $^V[D_4]_{LDS}$ and in step 2 mixing the four LDSs each at a variable volume of $^{vV}_{LDS(1)}$, $^{vV}_{LDS(2)}$, $^{vV}_{LDS(3)}$ and $^{vV}_{LDS(4)}$ to provide a LPC with a target concentration of all four serotypes of dengue virus of $^T[D_1]_{LPC}$, $^T[D_2]_{LPC}$, $^T[D_3]_{LPC}$, $^T[D_4]_{LPC}$.

Item 16: The batch mixing process of any one of the preceding items, wherein VEL, LDS(s), FEL and LPC are all aqueous liquids with a pH of from pH 7.0 to pH 8.5.

Item 17: The batch mixing process of any one of the preceding items, wherein the process further comprises:
step 3:

- drying the liquid pharmaceutical composition (LPC) obtained from step 2, such as by lyophilization, to obtain a dry pharmaceutical composition (DPC).

Item 18: A batch mixing process for preparing a liquid pharmaceutical composition (LPC) with a predefined target volume ($^TV_{LPC}$), comprising

(a) at least one adjustable excipient, wherein the at least one adjustable excipient has a target concentration in the LPC ($^T[E_x]_{LPC}$), and
(b) four biological active agents $A_1$, $A_2$, $A_3$ and $A_4$,

wherein said four biological active agents are

- dengue serotype 1 (dengue-1)
- dengue serotype 2 (dengue-2)
- dengue serotype 3 (dengue-3)
- dengue serotype 4 (dengue-4)

and wherein each biological active agent has a target concentration in the LPC ($^T[A_1]_{LPC}$, $^T[A_2]_{LPC}$, $^T[A_3]_{LPC}$, $^T[A_4]_{LPC}$),
wherein said batch mixing process comprises the following steps:

(i) providing four liquid drug substances (LDS), each LDS comprising a biological active agent at variable concentration ($^V[A_1]_{LDS1}$, $^V[A_2]_{LDS2}$, $^V[A_3]_{LDS3}$, $^V[A_4]_{LDS4}$)
(ii) combining variable volumes ($^{vV}_{LDS(i)}$) of the four LDSs together making a combined total volume of all LDSs according to equation 6:

$$^vV_{LDS(total)} = \ ^vV_{LDS(1)} + \ ^vV_{LDS(2)} + \ ^vV_{LDS(3)} + \ ^vV_{LDS(4)} \tag{6}$$

and either subsequently to step (ii) or simultaneously with step (ii);
(iii) combining an intermediate liquid (IL) at a variable volume $^{vV}_{IL}$, with said combined total volume of all LDSs $^vV_{LDS(total)}$ to generate the LPC;

wherein said batch-mixing process is characterised in that:

- the variable volume ($^{vV}_{LDS(i)}$) of each of the four LDSs is defined in accordance to equation 2:

$$^vV_{LDS(i)} = \frac{^T[Ai]_{LPC} \times \ ^TV_{LPC}}{^v[Ai]_{LDS(i)}} \tag{2}$$

- the variable volume of intermediate liquid (IL) $^{vV}_{IL}$ is calculated by subtracting the calculated combined total volume of all LDSs to be combined in step (ii) from the predefined target volume of the LPC according to the equation 3:

$$^vV_{IL} = \ ^TV_{LPC} - \ ^vV_{LDS(total)} \tag{3}$$

- the IL is made by mixing a predefined volume of a fixed excipient liquid (FEL) $^pV_{FEL}$ with a variable volume of variable excipient liquid (VEL) $^{vV}_{VEL}$, said variable volume of VEL being calculated by subtracting the predefined volume of the FEL from the calculated variable volume of IL $^{vV}_{IL}$ according to the equation 4:

$$^vV_{VEL} = \ ^vV_{IL} - \ ^pV_{FEL} \tag{4}$$

wherein the adjustable excipient(s) in the VEL are at the same concentration(s) as the adjustable excipient(s) in the combined total volume of all LDSs.

Item 19: The batch mixing process of item 18, wherein the LPC further comprises (c) at least one set excipient.

Item 20: The batch mixing process of item 19, wherein the adjustable excipients are phosphate buffer, sodium chloride, potassium chloride, and trehalose, and the set excipients are F127 and HSA.

Item 21: The batch mixing process of any one of items 18 to 20, wherein the process further comprises:
step 3:

- drying the liquid pharmaceutical composition (LPC), such as by lyophilization, to obtain a dry pharmaceutical composition (DPC).

Item 22: The batch mixing process of any one of the preceding items, wherein the batch mixing process is a large-scale batch process.

Item 23: A pharmaceutical composition (PC) obtainable by the batch mixing process according to any of the items above.

Item 24: The pharmaceutical composition (PC) of item 18, for use in a method of treatment or prevention, in particular prevention, of a disease in a subject or subject population.

Item 25: The pharmaceutical composition (PC) of item 18, for use in a method of treatment or prevention, in particular prevention of dengue disease, in a subject or subject population.

**Claims**

1. A batch mixing process for preparing a liquid pharmaceutical composition (LPC) comprising at least one biological active agent and at least one adjustable excipient, wherein the at least one biological active agent has a target concentration in the LPC ($^T[A_i]_{LPC}$) and the at least one adjustable excipient has a target concentration in the LPC ($^T[E_x]_{LPC}$), the process comprising at least two steps:

   step 1: providing

   • a variable excipient liquid (VEL) comprising the at least one adjustable excipient at a predefined concentration ($^P[E_x]_{VEL}$),
   • one or more liquid drug substances (LDS) comprising one biological active agent at a variable concentration ($^V[A_i]_{LDS}$) and the at least one adjustable excipient at a predefined concentration ($^P[E_x]_{LDS}$), and
   • a fixed excipient liquid (FEL) comprising the at least one adjustable excipient at a predefined concentration ($^P[E_x]_{FEL}$), wherein the predefined concentration of each of the adjustable excipients is defined in accordance with equation 1:

   $$^P[E_x]_{VEL} = {}^P[E_x]_{LDS} \neq {}^P[E_x]_{FEL} \qquad (1)$$

   wherein $^P[E_x]_{LDS}$ is predefined by the process of manufacture of LDS and $^P[E_x]_{FEL}$ is further defined by the conditions of step 2
   step 2: combining

   • all of the LDSs each at a variable volume ($^vV_{LDS(i)}$),
   • the VEL at a variable volume ($^vV_{VEL}$), and
   • the FEL at a predefined volume ($^PV_{FEL}$),

   to form the LPC with a target volume ($^TV_{LPC}$) and with a target concentration of all of the biological active agents and all of the adjustable excipients, wherein
   the variable volume of each LDS is defined according to equation 2:

   $$^vV_{LDS(i)} = \frac{^T[A_i]_{LPC}}{^V[A_i]_{LDS}} \times {}^TV_{LPC}, \qquad (2)$$

   the combined total volume ($^PV_{(VEL+LDS)}$) of all LDSs ( $^VV_{LDS(total)} = \sum_{i=1}^{\infty} V_{LDS(i)}$ ) and the VEL is predefined, and the variable volume of the VEL is defined according to equation 3:

   $$^vV_{VEL} = {}^PV_{(VEL+LDS)} - {}^vV_{LDS(total)}, \qquad (3)$$

   and the predefined volume of the FEL ($^PV_{FEL}$) is defined according to equation 4:

   $$^pV_{FEL} = {}^TV_{LPC} - {}^pV_{(VEL+LDS)}, \qquad (4)$$

   and the pre-defined concentration of each of the adjustable excipients in the FEL is defined according to equation 5:

$$^{p}[E_x]_{FEL} = \frac{^{T}[E_x]_{LPC} \times {}^{T}V_{LPC} - {}^{p}V_{(VEL+LDS)} \times {}^{p}[E_x]_{VEL}}{{}^{p}V_{FEL}} \qquad (5)$$

2. The batch mixing process of claim 1, comprising in step 1

- providing one LDS comprising one biological active agent at a variable concentration of $^{V}[A_1]_{LDS}$, and in step 2 mixing the one LDS at a variable volume of $^{V}V_{LDS(1)}$ to provide a LPC with a target concentration of one biological active agent of $^{T}[A_1]_{LPC}$;
- or providing two LDSs each comprising one biological active agent at a variable concentration of $^{V}[A_1]_{LDS}$ and $^{V}[A_2]_{LDS}$, and in step 2 mixing the two LDSs each at a variable volume of $^{V}V_{LDS(1)}$ and $^{V}V_{LDS(2)}$ to provide a LPC with a target concentration of the two biological active agents of $^{T}[A_1]_{LPC}$ and $^{T}[A_2]_{LPC}$; or
- providing three LDSs each comprising one biological active agent at a variable concentration of $^{V}[A_1]_{LDS}$, $^{V}[A_2]_{LDS}$ and $^{V}[A_3]_{LDS}$ and in step 2 mixing the three LDSs each at a variable volume of $^{V}V_{LDS(1)}$, $^{V}V_{LDS(2)}$ and $^{V}V_{LDS(3)}$ to provide a LPC with a target concentration of the three biological active agents of $^{T}[A_1]_{LPC}$, $^{T}[A_2]_{LPC}$. and $^{T}[A_3]_{LPC}$; or
- providing four LDSs each comprising one biological active agent at a variable concentration of $^{V}[A_1]_{LDS}$, $^{V}[A_2]_{LDS}$, $^{V}[A_3]_{LDS}$ and $^{V}[A_4]_{LDS}$ and in step 2 mixing the four LDSs each at a variable volume of $^{V}V_{LDS(1)}$, $^{V}V_{LDS(2)}$, $^{V}V_{LDS(3)}$ and $^{V}V_{LDS(4)}$ to provide a LPC with a target concentration of the four biological active agents of $^{T}[A_1]_{LPC}$, $^{T}[A_2]_{LPC}$, $^{T}[A_3]_{LPC}$ and $^{T}[A_4]_{LPC}$.

3. The batch mixing process according to any one of the preceding claims, wherein in step 2 the component liquids are combined in the following order:

   a. the FEL and the VEL are first combined to provide an intermediate liquid (IL),
   b. the IL and the at least one LDS are then subsequently combined to provide the LPC.

4. The batch mixing process of any one of the preceding claims for preparing a LPC, wherein

   the VEL, LDS(s), and FEL each comprise a predefined concentration of one adjustable excipient

   - $^{P}[E_a]_{VEL}$ in the VEL,
   - $^{P}[E_a]_{LDS}$ in the LDS and
   - $^{P}[E_a]_{FEL}$ in the FEL,

   providing a LPC with a target concentration of the one adjustable excipient $^{T}[E_a]_{LPC}$; or wherein
   the VEL, LDS(s), and FEL each comprise a predefined concentration of two adjustable excipients

   - $^{P}[E_a]_{VEL}$ and $^{P}[E_b]_{VEL}$ in the VEL,
   - $^{P}[E_a]_{LDS}$ and $^{P}[E_b]_{LDS}$ in the LDS and
   - $^{P}[E_a]_{FEL}$ and $^{P}[E_b]_{FEL}$ in the FEL,

   providing a LPC with a target concentration of the two adjustable excipients $^{T}[E_a]_{LPC}$ and $^{T}[E_b]_{LPC}$; or wherein
   the VEL, LDS(s), and FEL each comprise a predefined concentration of three adjustable excipients

   - $^{P}[E_a]_{VEL}$, $^{P}[E_b]_{VEL}$ and $^{P}[E_c]_{VEL}$ in the VEL,
   - $^{P}[E_a]_{LDS}$, $^{P}[E_b]_{LDS}$ and $^{P}[E_c]_{LDS}$ in the LDS and
   - $^{P}[E_a]_{FEL}$, $^{P}[E_b]_{FEL}$ and $^{P}[E_c]_{FEL}$ in the FEL;

   providing a LPC with a target concentration of the three adjustable excipients $^{T}[E_a]_{LPC}$, $^{T}[E_b]_{LPC}$ and $^{T}[E_c]_{LPC}$; or wherein
   the VEL, LDS(s), and FEL each comprise a predefined concentration of four adjustable excipients

   - $^{P}[E_a]_{VEL}$, $^{P}[E_b]_{VEL}$, $^{P}[E_c]_{VEL}$ and $^{P}[E_d]_{VEL}$ in the VEL,
   - $^{P}[E_a]_{LDS}$, $^{P}[E_b]_{LDS}$, $^{P}[E_c]_{LDS}$ and $^{P}[E_d]_{LDS}$ in the LDS and
   - $^{P}[E_a]_{FEL}$, $^{P}[E_b]_{FEL}$, $^{P}[E_c]_{FEL}$ and $^{P}[E_d]_{FEL}$ in the FEL;

providing a LPC with a target concentration of the four adjustable excipients ${}^T[E_a]_{LPC}$, ${}^T[E_b]_{LPC}$, ${}^T[E_c]_{LPC}$ and ${}^T[E_d]_{LPC}$.

5. The batch mixing process of any one of the preceding claims, wherein the VEL, LDS(s), and FEL further comprise at least one set excipient; wherein optionally the at least one set excipient is selected from the group of non-ionic surfactants and albumins.

6. The batch mixing process of claim 5, wherein the VEL, LDS(s), FEL each comprise a predefined concentration of four adjustable excipients

- ${}^P[E_a]_{VEL}$, ${}^P[E_b]_{VEL}$, ${}^P[E_c]_{VEL}$ and ${}^P[E_d]_{VEL}$ in the VEL,
- ${}^P[E_a]_{LDS}$, ${}^P[E_b]_{LDS}$, ${}^P[E_c]_{LDS}$ and ${}^P[E_d]_{LDS}$ in the LDS and
- ${}^P[E_a]_{FEL}$, ${}^P[E_b]_{FEL}$, ${}^P[E_c]_{FEL}$ and ${}^P[E_d]_{FEL}$ in the FEL; and
a predefined concentration of two set excipients,
providing a LPC with a target concentration of four adjustable excipients ${}^T[E_a]_{LPC}$, ${}^T[E_b]_{LPC}$, ${}^T[E_c]_{LPC}$ and ${}^T[E_d]_{LPC}$; and
two set excipients.

7. The batch mixing process of any one of the previous claims, wherein the one biological active agent is a virus, such as a live attenuated virus.

8. The batch mixing process of claim 7, wherein the virus is a flavivirus, such as a dengue virus.

9. The batch mixing process of claim 8, comprising in step 1 providing four LDSs each comprising one of the four serotypes of dengue virus $D_1$ $D_2$ $D_3$ and $D_4$ at a variable concentration of ${}^V[D_1]_{LDS}$, ${}^V[D_2]_{LDS}$, ${}^V[D_3]_{LDS}$, and ${}^V[D_4]_{LDS}$ and in step 2 mixing the four LDSs each at a variable volume of ${}^{VV}V_{LDS(1)}$, ${}^{VV}V_{LDS(2)}$, ${}^{VV}V_{LDS(3)}$ and ${}^{VV}V_{LDS(4)}$ to provide a LPC with a target concentration of all four serotypes of dengue virus of ${}^T[D_1]_{LPC}$, ${}^T[D_2]_{LPC}$, ${}^T[D_3]_{LPC}$, ${}^T[D_4]_{LPC}$.

10. The batch mixing process of any one of the preceding claims, wherein the process further comprises:
step 3:

• drying the liquid pharmaceutical composition (LPC) obtained from step 2, such as by lyophilization, to obtain a dry pharmaceutical composition (DPC).

11. A batch mixing process for preparing a liquid pharmaceutical composition (LPC) with a predefined target volume (${}^TV_{LPC}$), comprising

(c) at least one adjustable excipient, wherein the at least one adjustable excipient has a target concentration in the LPC (${}^T[E_x]_{LPC}$), and
(d) four biological active agents $A_1$, $A_2$, $A_3$ and $A_4$,
wherein said four biological active agents are

- dengue serotype 1 (dengue-1)
- dengue serotype 2 (dengue-2)
- dengue serotype 3 (dengue-3)
- dengue serotype 4 (dengue-4)

and wherein each biological active agent has a target concentration in the LPC (${}^T[A_1]_{LPC}$, ${}^T[A_2]_{LPC}$, ${}^T[A_3]_{LPC}$, ${}^T[A_4]_{LPC}$),
wherein said batch mixing process comprises the following steps:

(iv) providing four liquid drug substances (LDS), each LDS comprising a biological active agent at variable concentration (${}^V[A_1]_{LDS1}$, ${}^V[A_2]_{LDS2}$, ${}^V[A_3]_{LDS3}$, ${}^V[A_4]_{LDS4}$)
(v) combining variable volumes (${}^{VV}V_{LDS(i)}$) of the four LDSs together making a combined total volume of all LDSs according to equation 6:

$$ {}^vV_{LDS(total)} = {}^vV_{LDS(1)} + {}^vV_{LDS(2)} + {}^vV_{LDS(3)} + {}^vV_{LDS(4)} \qquad (6) $$

and either subsequently to step (ii) or simultaneously with step (ii);

(vi) combining an intermediate liquid (IL) at a variable volume $^vV_{IL}$, with said combined total volume of all LDSs $^vV_{LDS(total)}$ to generate the LPC;

wherein said batch-mixing process is **characterised in that**:

• the variable volume ($^{v}V_{LDS(i)}$) of each of the four LDSs is defined in accordance to equation 2:

$$^vV_{LDS(i)} = \frac{^T[Ai]_{LPC} \times \, ^TV_{LPC}}{^v[Ai]_{LDS(i)}} \qquad (2)$$

• the variable volume of intermediate liquid (IL) $^vV_{IL}$ is calculated by subtracting the calculated combined total volume of all LDSs to be combined in step (ii) from the predefined target volume of the LPC according to the equation 3:

$$^vV_{IL} = \, ^TV_{LPC} - \, ^vV_{LDS(total)} \qquad (3)$$

• the IL is made by mixing a predefined volume of a fixed excipient liquid (FEL) $^pV_{FEL}$ with a variable volume of variable excipient liquid (VEL) $^vV_{VEL}$, said variable volume of VEL being calculated by subtracting the predefined volume of the FEL from the calculated variable volume of IL $^vV_{IL}$ according to the equation 4:

$$^vV_{VEL} = \, ^vV_{IL} - \, ^pV_{FEL} \qquad (4)$$

wherein the adjustable excipient(s) in the VEL are at the same concentration(s) as the adjustable excipient(s) in the combined total volume of all LDSs.

12. The batch mixing process of claim 11, wherein the process further comprises:
step 3:

• drying the liquid pharmaceutical composition (LPC), such as by lyophilization, to obtain a dry pharmaceutical composition (DPC).

13. The batch mixing process of any one of the preceding claims, wherein the batch mixing process is a large-scale batch process.

14. A pharmaceutical composition (PC) obtainable by the batch mixing process according to any of the claims above.

15. The pharmaceutical composition (PC) of claim 14, for use in a method of treatment or prevention, in particular prevention of dengue disease, in a subject or subject population.

Figure 1:

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63310437
- WO 9837911 A
- WO 03101397 A
- WO 2007021672 A
- WO 2008007021 A
- WO 2008047023 A
- WO 2008065315 A
- WO 01060847 A2
- WO 2014150939 A2
- WO 2017179017 A1

**Non-patent literature cited in the description**

- **BHAMARAPRAVATI et al.** *Bulletin of the World Health Organization*, 1987, vol. 65 (2), 189-195
- **XIAO et al.** *Exp. Neurobiol.*, 1997, vol. 144, 113-124
- **FISHER et al.** *J. Virol.*, 1996, vol. 70, 520-532
- **BREWOO et al.** *Vaccine*, 14 February 2012, vol. 30 (8), 1513-1520